# EUROPÄISCHE PATENTSCHRIFT

(11) **EP 4 262 889 B1**
(45) Veröffentlichungstag und Bekanntmachung des Hinweises auf die Patenterteilung: **28.01.2026**
(21) Anmeldenummer: 21840491.1
(22) Anmeldetag: 15.12.2021
(51) Int. Cl.: A61L 2/00, A61M 1/36

(54) **MODULARE BESTRAHLUNGSVORRICHTUNG UND BESTRAHLUNGSVERFAHREN**
MODULAR IRRADIATION DEVICE AND IRRADIATION METHOD
DISPOSITIF D'IRRADIATION MODULAIRE ET PROCÉDÉ D'IRRADIATION

(30) Priorität: 16.12.2020 DE 102020216088
(43) Veröffentlichungstag der Anmeldung: 25.10.2023
(73) Patentinhaber: Fraunhofer-Gesellschaft zur Förderung der angewandten Forschung e.V., 80686 München (DE)
(72) Erfinder: STANDFEST, Bastian, 70569 Stuttgart (DE); THOMA, Martin, 70569 Stuttgart (DE)
(74) Vertreter: Pfenning, Meinig & Partner mbB
(86) Internationale Anmeldenummer: PCT/EP2021/085979
(87) Internationale Veröffentlichungsnummer: WO 2022/129219

(56) Entgegenhaltungen:
- WO-A1-2022/084656
- DE-A1- 10 053 441
- US-A1- 2003 030 011
- US-A1- 2013 138 037
- US-B2- 9 816 073

## Beschreibung

Die Erfindung betrifft eine modulare Bestrahlungsvorrichtung, die ein Hauptmodul und zumindest eine Trägerkassette aufweist, wobei die Trägerkassette in eine Aufnahme des Hauptmoduls einsetzbar ist. Dabei weist die Trägerkassette zumindest eine Pumpe auf und das Hauptmodul zumindest einen Pumpenaktor auf, die so angeordnet sind, dass die Pumpe mit dem Pumpenaktor aktuierbar ist, wenn die Trägerkassette in die Aufnahme des Hauptmoduls eingesetzt ist.

Sowohl bei Herstellung von Arzneimittel für neuartige Therapien (ATMPs) und personalisierter Medizin als auch bei der Inaktivierung und Sterilisation von bspw. pathogenen Flüssigkeiten kommt vermehrt ionisierende Strahlung zum Einsatz. Um eine homogene Bestrahlung zu gewährleisten, muss ein konstanter Dosiseintrag in die Flüssigkeit sichergestellt werden. Beispielsweise bei der Bestrahlung mit niederenergetischer Elektronenstrahlung verlieren die beschleunigten Elektronen mit zunehmender Eindringtiefe an Energie, so dass die Tiefendosis abnimmt. Dies hat zur Folge, dass der zu behandelnde Flüssigkeitsfilm eine Schichtdicke von <200µm aufweisen soll. Neben gleichbleibenden Strahlenparameter sind außerdem konstante Fließeigenschaften des Flüssigkeitsfilms von Bedeutung, welche sich unter anderem aus Schichtdicke und Fließgeschwindigkeit zusammensetzen.

Ein weiterer wichtiger Aspekt bei der Prozessierung von ATMPs ist die Vermeidung von Querkontaminationen. Vor allem hinsichtlich der Produktion personalisierter Medizin und/oder der Handhabung von kleinvolumigen Patientenproben ist der Gebrauch von standardisierten Disposables wie beispielsweise Spritzen, Infusionsbeutel, etc. vorteilhaft.

Die DE102016216573A1 beschreibt die Bestrahlung von dünnen Flüssigkeitsschichten mit Hilfe einer sich drehenden Edelstahlrolle. Da es sich hierbei um eine produktberührende Lösung handelt, kann ein schneller, querkontaminationsfreier Tausch zwischen verschiedenen Patientenproben nicht gewährleistet werden.

DE 100 53 441 A1 offenbart eine Vorrichtung zur Zuführung und/oder Abzweigung einer Nebenströmung in eine und/oder aus einer Hauptströmung einer medizinischen Flüssigkeit.

US 2013/138037 A1 offenbart ein System zum automatischen Verbinden von Schläuchen unter Beibehaltung der Sterilität. Das System umfasst einen Rahmen zur Montage neben einer Kassette in einer Dialysemaschine und einen im Rahmen montierten Shuttle, der zur Aufnahme von Schläuchen aus mindestens zwei Behältern konfiguriert ist.

US 9 816 073 B2 offenbart eine Vorrichtung zur Bestrahlung biologischer Flüssigkeiten, wie Blut und Blutbestandteile, in einem in einer Behandlungskammer angeordneten Behälter.

US 2003/030011 A1 offenbart ein Fluidbehandlungssystem unter Verwendung von Lichtimpulsen als Lichtbehandlung, welche der Inaktivierung von Mikroorganismen, einschließlich Viren, Bakterien, Pilzen und anderen pathogenen und nicht-pathogenen Mikroorganismen dient.

Aufgabe der vorliegenden Erfindung ist es, eine Bestrahlungsvorrichtung und ein Bestrahlungsverfahren anzugeben, die eine effiziente Bestrahlung von Fluidproben bei gleichzeitig möglichst geringer Gefahr von Querkontamination ermöglichen.

Die Aufgabe wird gelöst durch die modulare Bestrahlungsvorrichtung nach Anspruch 1 und das Bestrahlungsverfahren nach Anspruch 19. Die jeweiligen abhängigen Ansprüche beschreiben vorteilhafte Weiterbildungen der erfindungsgemäßen modularen Bestrahlungsvorrichtung und des erfindungsgemäßen Bestrahlungsverfahrens.

Die Erfindung betrifft eine Bestrahlungsvorrichtung, die modular ist. Die Bestrahlungsvorrichtung weist als Module zum einen ein Hauptmodul und zum anderen zumindest eine Trägerkassette auf. Module sollen dabei vorzugsweise bauliche Einheiten sein, deren Bestandteile jeweils strukturell miteinander verbunden sind und gemeinsam gehandhabt werden können, ohne Bestandteile der Module lösen zu müssen.

Erfindungsgemäß weist das Hauptmodul zumindest eine Aufnahme auf, in welche die zumindest eine Trägerkassette zerstörungsfrei entfernbar einsetzbar ist. Vorzugsweise ist die Trägerkassette in die Aufnahme einsetzbar, ohne Bestandteile der Trägerkassette voneinander vereinzeln zu müssen und ohne Bestandteile des Hauptmoduls voneinander vereinzeln zu müssen. Hauptmodul und zumindest eine Trägerkassette sind also vorzugsweise so ausgestaltet, dass die Trägerkassette in die Aufnahme des Hauptmoduls einsetzbar ist in einem Zustand, in dem alle Bestandteile der Trägerkassette miteinander verbunden sind und in dem alle Bestandteile des Hauptmoduls miteinander verbunden sind und ohne dass ein oder mehrere Elemente der Trägerkassette vereinzelt werden müssten und ohne dass ein oder mehrere Elemente des Hauptmoduls vereinzelt werden müssten. Es kann als Ausdruck der modularen Idee der Erfindung gesehen werden, dass die verschiedenen Module, also insbesondere Hauptmodul und Trägerkassette, als Ganzes gehandhabt werden können.

Erfindungsgemäß weist die Trägerkassette zumindest eine Expositionsoberfläche auf, auf der zumindest eine Bestrahlungsleitung verläuft. Der Begriff Expositionsoberfläche soll hierbei zunächst einfach als Fläche verstanden werden, die durch eine Bestrahlungsquelle bestrahlbar ist. Im einfachsten Fall könnte dies auch lediglich die Oberfläche der Bestrahlungsleitung selbst sein. Ist also beispielsweise die Bestrahlungsleitung ein Schlauch, so könnte die der Bestrahlungsquelle zugewandte Schlauchoberfläche als Expositionsoberfläche angesehen werden. Bevorzugt ist es jedoch, wenn die Expositionsoberfläche eine Fläche ist, in die die Bestrahlungsleitung eingebracht ist.

Erfindungsgemäß ist in der zumindest einen Bestrahlungsleitung ein zu bestrahlendes Fluid leitbar. Das Fluid kann zum Beispiel ein Gas oder vorzugsweise eine Flüssigkeit sein, wobei Suspensionen, beispielsweise von Zellen, ebenfalls als Flüssigkeit in diesem Sinne angesehen werden können. Die Fluidleitung kann beispielsweise ein Schlauch oder ein Kanal sein, wobei ein Kanal in die Expositionsoberfläche eingebracht und durch eine die Expositionsoberfläche zumindest teilweise bedeckende Folie bedeckt sein kann.

Erfindungsgemäß weist die Trägerkassette zumindest eine Pumpe auf, die über eine erste Fluidleitung an der zumindest einen Bestrahlungsleitung angeschlossen ist. Unter einer Pumpe kann hier eine Vorrichtung verstanden werden, mit der das Fluid förderbar ist. Vorzugsweise werden dabei jene Elemente als Pumpe bezeichnet, die auf das Fluid wirken, also insbesondere beispielsweise ein Kolben und eine Pumpenkammer, jedoch soll ein Aktor zur Aktuierung der Pumpe nicht zur Pumpe selbst gerechnet werden.

Dass die zumindest eine Pumpe über die erste Fluidleitung an der zumindest einen Bestrahlungsleitung angeschlossen ist, bedeutet hier, dass durch Aktuierung der Pumpe Fluid durch die erste Fluidleitung in die Bestrahlungsleitung bewegbar ist oder aus der Bestrahlungsleitung in die erste Fluidleitung bewegbar ist. Die Pumpe ist also über die erste Fluidleitung fluidleitend an der zumindest einen Bestrahlungsleitung angeschlossen.

Erfindungsgemäß weist das Hauptmodul zumindest eine Aufnahme auf, in welche die zumindest eine Trägerkassette zerstörungsfrei entfernbar einsetzbar ist. Besonders bevorzugt ist die zumindest eine Trägerkassette in die zumindest eine Aufnahme einsetzbar ohne Vereinzelung von Bestandteilen des Hauptmoduls und ohne Vereinzelung von Bestandteilen der Trägerkassette.

Die Trägerkassette ist also vorzugsweise als Ganzes in die Aufnahme einsetzbar.

Erfindungsgemäß weist das Hauptmodul außerdem zumindest einen Pumpenaktor auf, der so angeordnet ist, dass mit ihm die zumindest eine Pumpe der Trägerkassette aktuierbar ist, wenn die entsprechende Trägerkassette in der entsprechenden Aufnahme eingesetzt ist. Der Vorgang des Einsetzens der Trägerkassette in die Aufnahme kann mehrere Schritte umfassen, wie beispielsweise das Platzieren der Trägerkassette in der Aufnahme und das Schließen beispielsweise einer Verriegelung oder ähnliche weitere Schritte. Insbesondere kann der Vorgang des Einsetzens Schritte umfassen, in denen beispielsweise ein Pumpenkopplungselement des Pumpenaktors mit der Pumpe beziehungsweise einem aktuierbaren Element der Pumpe in Eingriff gebracht wird. Auch beispielsweise ein Einsetzen von Verbindungselementen zwischen dem Aktor und der entsprechenden Pumpe kann als Teil des Einsetzprozesses verstanden werden. Sofern diese Verbindungselemente nicht mit dem Hauptmodul oder der Trägerkassette verbunden sind, würden sie zu keinem der beiden Module gezählt werden. Die Aktuierbarkeit der Pumpe durch den Pumpenaktor sollte spätestens dann gegeben sein, wenn alle Schritte des Einsetzens der Trägerkassette in die Aufnahme abgeschlossen sind.

Es gibt eine große Anzahl verschiedener Möglichkeiten, die zumindest eine Pumpe in der Trägerkassette und den zumindest einen Pumpenaktor am Hauptmodul anzuordnen, so dass die Pumpe mit dem Pumpenaktor aktuierbar ist, wenn die Trägerkassette in die Aufnahme des Hauptmoduls eingesetzt ist. Die genaue Ausgestaltung hängt von der Ausgestaltung der Pumpe wie auch von der Ausgestaltung des Pumpenaktors ab. Ist beispielsweise die Pumpe als Spritze ausgestaltet, so kann der Pumpenaktor beispielsweise eine Druckfläche aufweisen, die beim Einsetzen der Trägerkassette in die Aufnahme an einer Endfläche eines Spritzenkolbens der Spritze zum Anliegen kommt, so dass der Pumpenaktor mit der Druckfläche auf die Endfläche des Spritzenkolbens drücken kann und dadurch den Spritzenkolben in den Spritzenzylinder drücken kann. Alternativ oder zusätzlich kann der Pumpenaktor beispielsweise auch ein Greifelement aufweisen, das hinter der Endfläche des Pumpenkolbens an deren Rückseite anliegen kann, wenn die Kassette in der Aufnahme eingesetzt ist, so dass der Pumpenaktor den Kolben aus dem Spritzenzylinder herausziehen kann. Diese Ausgestaltungen sollen jedoch lediglich als Beispiele verstanden werden. Bei einer gegebenen Pumpenform wird es einem Fachmann immer möglich sein, den Aktor entsprechend zu wählen und anzuordnen, dass dieser die Pumpe aktuieren kann.

In einer vorteilhaften Ausgestaltung der Erfindung kann die Trägerkassette zumindest eine weitere der Pumpen aufweisen, die über eine zweite Fluidleitung an der Bestrahlungsleitung angeschlossen ist. Insbesondere kann eine solche weitere Pumpe vorteilhaft an einem gegenüberliegenden Ende der Bestrahlungsleitung gegenüber der ersten Pumpe angeordnet sein. Auf diese Weise kann das Fluid von der einen Pumpe durch die Bestrahlungsleitung zu der anderen Pumpe geleitet werden. Es ist dabei ausreichend, wenn nur eine der Pumpen oder nur die Pumpen auf nur einer Seite der Bestrahlungsleitung aktuiert sind. Die Pumpen der anderen Seite wirken dann lediglich als Bereitstellungs- oder Empfangsbehältnis und werden durch das Fluid selbst bewegt. Vorteilhaft ist eine Ausgestaltung, in der jene Pumpen aktuiert werden, in die das Fluid durch die Bestrahlungsleitung hindurch bewegt wird. In dem Fall wird also das Fluid durch die Bestrahlungsleitung gesaugt. Vorteilhaft ist hierbei, dass das Fluid nicht durch undichte Stelle aus dem System austreten kann. Es ist auch möglich, die Fluide mit Überdruck zu transportieren.

In einer vorteilhaften Ausgestaltung der Erfindung kann der Pumpenaktor ein Pumpenkopplungselement aufweisen, das so angeordnet ist, dass es mit einem beweglichen Element der entsprechenden Pumpe formschlüssig in Eingriff kommt, wenn die Kassette in das Hauptmodul eingesetzt wird. Es entsteht hierbei also ein Formschluss zwischen dem Pumpenkopplungselement und dem beweglichen Element der Pumpe, über den der Pumpenaktor eine Kraft oder ein Drehmoment auf das bewegliche Element der Pumpe ausüben kann. Das Pumpenkopplungselement kann im einfachsten Fall eine Anlage oder Druckfläche sein, die auf das bewegliche Element der Pumpe drücken kann, wenn der Aktor aktiv wird. Das bewegliche Element der Pumpe kann jedoch beispielsweise auch einen Überstand aufweisen, wobei dann das Pumpenkopplungselement beispielsweise einen Vorstand oder eine Gabel aufweisen kann, die hinter den Überstand greift, so dass das bewegliche Element der Pumpe in Richtung des Überstands aktuierbar ist. Auch möglich ist beispielsweise, dass das Pumpenkopplungselement und das bewegliche Element der Pumpe eine Verbindung wie beispielsweise eine Schwalbenschwanzverbindung eingehen, wenn die Trägerkassette in die Aufnahme des Hauptmoduls eingesetzt wird.

In einer vorteilhaften Ausgestaltung der Erfindung kann die zumindest eine Trägerkassette zumindest ein in zumindest einer der Fluidleitungen angeordnetes Ventil aufweisen, mit dem ein Fluidfluss zwischen der entsprechenden Pumpe, die über diese Fluidleitung an der Bestrahlungsleitung angeschlossen ist, und der Bestrahlungsleitung steuerbar ist. Dass das Ventil in der Fluidleitung angeordnet ist, bedeutet dabei, dass das Ventil an einem Ende der Fluidleitung angeordnet ist oder zwischen zwei Teilen der Fluidleitung angeschlossen ist, wobei dann ein Teil der Fluidleitung an einem Anschluss des Ventils angeordnet ist und der andere Teil der Fluidleitung an einem anderen Anschluss des Ventils angeordnet ist. Der Fluidfluss durch die Fluidleitung fließt also durch das Ventil.

In dieser Ausgestaltung weist vorzugsweise das Hauptmodul für eines, mehrere oder alle der Ventile jeweils einen Ventilaktor auf, der oder die jeweils so angeordnet sind, dass durch sie das entsprechende Ventil verstellbar ist, wenn die Trägerkassette in der entsprechenden Aufnahme des Hauptmoduls eingesetzt ist. Auf diese Weise kann durch Einsetzen der Trägerkassette in die Aufnahme ein Zustand hergestellt werden, in dem die Ventile durch die Ventilaktoren aktuiert werden können. Die Ventilaktoren können dann vorteilhaft automatisch gesteuert werden, so dass über die Ventile der Fluidfluss in der Trägerkassette automatisch steuerbar ist.

Der zumindest eine Pumpenaktor und/oder der zumindest eine Ventilaktor können vorteilhaft elektrische Aktoren, pneumatische, hydraulische, magnetische Aktoren sein.

Vorteilhaft ist eine Ausgestaltung, in der das zumindest eine Ventil einen Hahn aufweist, mit dem es zur Steuerung des Fluidflusses verstellbar ist. In diesem Fall kann dann der entsprechende Ventilaktor, der bei Einsetzen der Trägerkassette in die Aufnahme mit diesem Ventil in Kontakt kommt, mit dem Hahn so koppelbar sein, dass mit dem Ventilaktor eine das Ventil verstellende Kraft oder ein das Ventil verstellendes Drehmoment auf den Hahn ausübbar ist. Vorteilhaft kann dabei zwischen dem Kopplungselement und dem Hahn des entsprechenden Ventils ein Formschluss entstehen, wenn die Trägerkassette in die Aufnahme eingesetzt wird. Vorteilhaft kann dann das Kopplungselement mit dem Hahn des entsprechenden Ventils in Eingriff kommen, wenn die Trägerkassette in das Hauptmodul eingesetzt wird. Auch hier gibt es zahlreiche Möglichkeiten, wie die Ventile mit ihren Hähnen mit den Kopplungselementen gekoppelt werden können. Diese Ausführungsform der Erfindung ist dabei nicht auf eine bestimmte Form der Kopplung oder eine bestimmte Anordnung beschränkt. Beispielsweise können die Ventile in der Trägerkassette so angeordnet sein, dass die Ventile außen an der Trägerkassette angeordnet sind und die Ventilhähne von der Trägerkassette weg nach außen gerichtet sind. Es können dann Ventilaktoren am Hauptmodul einen Bereich umgebend angeordnet sein, in dem die Trägerkassette vorliegt, wenn sie in die Aufnahme eingesetzt ist. Ist im eingesetzten Zustand jeweils der Ventilhahn auf der gleichen Höhe und in der gleichen Richtung angeordnet wie der entsprechende Aktor, so können Ventilhahn und Aktor miteinander in Eingriff kommen. Diese Ausgestaltung ist jedoch lediglich als Beispiel zu verstehen und andere Anordnungen sind ohne weiteres möglich.

In einer vorteilhaften Ausgestaltung der Erfindung kann zumindest eines der zumindest einen Ventile ein Dreiwegeventil sein, das drei Anschlüsse aufweist. Es kann dann vorteilhaft einer der drei Anschlüsse an einer der Fluidleitungen angeschlossen sein, die in der Bestrahlungsleitung münden und die anderen beiden Anschlüsse können jeweils mit einer Pumpe verbunden sein. Auf diese Weise erlaubt das Dreiwegeventil es, umzuschalten zwischen einem Zustand, in dem eine Pumpe mit der Bestrahlungsleitung verbunden ist und einem Zustand, in dem die andere Pumpe mit der Bestrahlungsleitung verbunden ist. Auf diese Weise können in der Trägerkassette mehrere Pumpen mit beispielsweise unterschiedlichen Fluiden vorgesehen sein, die zu unterschiedlichen Zeiten durch die Bestrahlungsleitung leitbar sind und zwischen denen durch Stellen des Ventils umgeschaltet werden kann.

Die Erfindung ist grundsätzlich mit jeder Art von Pumpe realisierbar. Bevorzugt ist jedoch eine Ausgestaltung, in der die zumindest eine Pumpe eine Fluidkammer und einen Kolben aufweist, wobei der Kolben die Fluidkammer fluiddicht abschließt und in der Fluidkammer verschiebbar ist. Insbesondere kann hierbei vorteilhafterweise die Fluidkammer zylinderförmig sein und der Kolben eine Stirnfläche haben, mit der er die Fluidkammer begrenzt, und deren Form im Wesentlichen identisch zur Grundfläche der Fluidkammer ist. Es kann der jeweilige Pumpenaktor am Kolben der Pumpe angreifen, wenn die zumindest eine Trägerkassette in der entsprechenden Aufnahme eingesetzt ist. Auf diese Weise kann mit dem Pumpenaktor eine Kraft in einer Verschiebungsrichtung des Kolben bewirkbar sein.

In einer vorteilhaften Ausgestaltung kann die Pumpe eine Spritze, besonders bevorzugt eine austauschbare Plastikspritze sein. Hierdurch wird es möglich, das oder die Fluid(e) vor oder nach der Bestrahlung in der entsprechenden Spritze unterzubringen und die Spritzen nach Gebrauch zu entsorgen. Hierdurch kann Sterilität des Systems hergestellt werden, da die Spritzen als kontaminierte Teile entsorgt werden können.

In einer besonders vorteilhaften Ausgestaltung der Erfindung kann die Trägerkassette zwei an der ersten Fluidleitung angeschlossene Pumpen aufweisen und drei an der zweiten Fluidleitung angeschlossene Pumpen. In dieser Ausgestaltung können beispielsweise in den drei an der zweiten Fluidleitung angeschlossenen Pumpen durch die Bestrahlungsleitung zu bewegende Fluide vorgesehen sein und die zwei an der ersten Fluidleitung angeschlossenen Pumpen können einerseits als Aufnahmegefäß für bestrahltes Fluid dienen und andererseits als Empfangsgefäß für Abfallprodukte.

In einer vorteilhaften Ausgestaltung kann in der ersten der an der zweiten Fluidleitung angeschlossenen Pumpen beispielsweise ein Desinfektionsmittel bereitgestellt werden, in der zweiten der drei Pumpen ein Zellmedium und in der dritten der drei Pumpen eine Zellsuspension. Beispielsweise über ein Dreiwegeventil kann dann zunächst die das Desinfektionsmittel aufweisende Pumpe mit der Bestrahlungsleitung verbunden werden und das Desinfektionsmittel durch die Bestrahlungsleitung gepumpt werden. Auf Seiten der ersten Fluidleitung kann, beispielsweise ebenfalls über ein Dreiwegeventil, die Spritze für Abfallprodukte mit der Bestrahlungsleitung verbunden sein. Das Desinfektionsmittel wird dann also durch die Bestrahlungsleitung in die Pumpe für Abfallprodukte befördert. Es kann dann auf Seiten der zweiten Fluidleitung, beispielsweise über ein Dreiwegeventil, die Spritze mit Zellmedium an die Bestrahlungsleitung angeschlossen werden und das Zellmedium durch die Bestrahlungsleitung transportiert werden. Auch dieses kann auf Seiten der ersten Fluidleitung beispielsweise in die Pumpe für Abfallprodukte befördert werden. Es kann dann anschließend auf Seiten der zweiten Fluidleitung die Pumpe mit Zellsuspension an die Bestrahlungsleitung angeschlossen werden und die Zellsuspension durch die Bestrahlungsleitung befördert werden, die dann beispielsweise einer Bestrahlung ausgesetzt werden kann. Auf Seiten der ersten Fluidleitung kann dann die Pumpe für bearbeitetes Fluid an die Bestrahlungsleitung angeschlossen werden, so dass die bestrahlte Zellsuspension in diese Pumpe befördert wird.

In einer vorteilhaften Ausgestaltung der Erfindung kann das Hauptmodul eine Hauptmoduloberfläche aufweisen, die gegenüber der Aufnahme so angeordnet ist, dass die Hauptmoduloberfläche und die Expositionsoberfläche der Trägerkassette koplanar sind, wenn die Trägerkassette in der entsprechenden Aufnahme des Hauptmoduls eingesetzt ist. Durch diese Hauptmoduloberfläche kann verhindert werden, dass Strahlung von der Bestrahlungsquelle auf andere Elemente des Hauptmoduls und/oder der Trägerkassette trifft, die nicht der Bestrahlung ausgesetzt werden sollen. Vorzugsweise sind die Expositionsoberfläche und die Hauptmoduloberfläche so ausgestaltet, dass nur die Bestrahlungsleitung der Bestrahlung ausgesetzt ist. Vorteilhafterweise kann die Expositionsoberfläche der Trägerkassette eine Öffnung in der Hauptmoduloberfläche, vorzugsweise vollständig, ausfüllen, so dass der innere Rand dieser Öffnung am äußeren Rand der Expositionsoberfläche anliegt.

In einer besonders vorteilhaften Ausgestaltung weist das Hauptmodul genau für jede jener Pumpen einen Aktor auf, die für die Aufnahme von Fluid vorgesehen sind, das die Bestrahlungsleitung durchlaufen hat. In dieser Ausgestaltung sind für jene Pumpen, in denen Fluid bereitgestellt wird, um durch die Bestrahlungsleitung geleitet zu werden, keine Aktoren vorgesehen. In dieser Ausgestaltung wird der Fluidtransport dadurch bewirkt, dass das Fluid durch die Bestrahlungsleitung gesaugt wird. Dies ist vorteilhaft, da ein Austreten von Fluid durch eventuelle undichte Stellen verhindert wird.

In einer vorteilhaften Ausgestaltung der Erfindung kann das Hauptmodul zwei klappbare Seitenteile aufweisen, mit denen vorteilhafterweise die Aufnahme für die zumindest eine Trägerkassette verschließbar ist. Es kann also die entsprechende Trägerkassette zunächst in die Aufnahme einsetzbar sein und der Vorgang des Einsetzens dadurch fortgesetzt oder abgeschlossen werden, dass die klappbaren Seitenteile in eine verschlossene Position geklappt werden.

Besonders vorteilhaft können dabei die klappbaren Seitenteile einige oder alle der Aktoren und/oder Pumpenaktoren aufweisen. Besonders bevorzugt ist es hier, wenn solche der Aktoren, mit denen die Pumpen und/oder Ventile, die über die erste Fluidleitung an der Bestrahlungsleitung angeschlossen sind, aktuierbar sind, an einem der Seitenteile angeordnet sind und solche der Aktoren, mit denen die Pumpen und/oder Ventile, die über die zweite Fluidleitung an der Bestrahlungsleitung angeschlossen sind, aktuierbar sind, am anderen der Seitenteile angeordnet sind. Diese Anordnung ist insbesondere sinnvoll, wenn die an der ersten und zweiten Fluidleitung angeschlossenen Pumpen auf gegenüberliegenden Hälften der Trägerkassette angeordnet sind. Die zwei Seitenteile können sich dann im geschlossenen Zustand an einer Ebene treffen, die die Bestrahlungsleitung durchschneidet, vorzugsweise halbiert, und die zwischen den an der ersten Fluidleitung angeschlossenen Pumpen und den an der zweiten Fluidleitung angeschlossenen Pumpen liegt.

In einer vorteilhaften Ausgestaltung der Erfindung kann die Trägerkassette einen Fluidchip aufweisen. Dieser Fluidchip kann einen Grundkörper aufweisen, der eine Grundkörperexpositionsoberfläche hat, in der die zumindest eine Bestrahlungsleitung ausgebildet sein kann. Die Grundkörperexpositionsoberfläche kann dabei die Expositionsoberfläche der Trägerkassette stellen oder Teil dieser Expositionsoberfläche sein. Vorteilhafterweise kann die zumindest eine Bestrahlungsleitung dann einen Kanal oder eine Kanalstruktur mit zumindest einem Kanal sein. Der Grundkörper kann einen ersten Fluidanschluss aufweisen, an den die erste Fluidleitung angeschlossen ist, sowie einen zweiten Fluidanschluss, an den die zweite Fluidleitung angeschlossen ist. Die Fluidanschlüsse können auf jede mögliche Weise ausgestaltet sein, beispielsweise als Steckverbindungen, aber auch eine Ausgestaltung, in die der Grundkörper und die erste und/oder zweite Fluidleitung monolithisch ausgestaltet sind, soll von dieser Ausgestaltung umfasst sein. Der Fluidanschluss wäre hier einfach der Übergang zwischen der entsprechenden Fluidleitung und der Kanalstruktur.

Der Grundkörper kann außerdem eine Folie aufweisen, welche auf der Grundkörperexpositionsoberfläche angeordnet ist und welche die Kanalstruktur bedeckt. Dabei kann die Folie die Kanalstruktur so bedecken, dass sie diese gegen Austreten von Fluid auf die Grundkörperexpositionsoberfläche abdichtet. Die Folie liegt auf der Expositionsoberfläche vorzugsweise zumindest in jeden Bereichen vor, wo die Kanalstruktur ausgebildet ist.

Als Grundkörper kann beispielsweise ein Polyethylenblock dienen, der beispielsweise spritzgegossen sein kann. Die Folie kann beispielsweise eine PET-PE-Folie oder Ähnliches aufweisen oder sein.

In einer vorteilhaften Ausgestaltung kann die Kanalstruktur eine Mehrzahl der Kanäle aufweisen, die an ihren jeweiligen Enden in den jeweiligen Fluidanschluss zusammenlaufen. Die Kanäle können also mit einem ihrer Enden an dem einen der Fluidanschlüsse zusammenlaufen und mit ihren anderen Enden an dem anderen der Fluidanschlüsse. Besonders vorteilhaft ist eine Ausgestaltung, in der die Kanäle an ihren Enden jeweils paarweise in gemeinsame Kanäle zusammenlaufen und die gemeinsamen Kanäle ihrerseits so oft jeweils paarweise in gemeinsame Kanäle zusammenlaufen, bis genau zwei gemeinsame Kanäle in einen der Fluidanschlüsse zusammenlaufen. Ausgehend vom jeweiligen Fluidanschluss kann sich hierbei eine Baumstruktur der Kanäle ergeben, in denen sich jeweils ein Kanal in zwei Kanäle aufspaltet, bis die sich aufspaltenden Kanäle in lange Kanäle münden, die über die Expositionsoberfläche laufen und auf der anderen Seite wiederum in eine Baumstruktur, vorzugsweise gleich ausgestaltet, münden, in der sie wieder zu dem anderen Fluidanschluss zusammengeführt werden. Die langen Abschnitte der Kanäle verlaufen dabei vorzugsweise gerade und parallel zueinander.

Der Grundkörper kann vorzugsweise ein monolithischer Block sein, beispielsweise ein Spritzgussteil, in den die Kanalstruktur eingeprägt und/oder eingeschnitten ist. Unter Einschneiden soll hierbei jede spanende Bearbeitung verstanden werden, also beispielsweise Schnitzen, Gravieren, Fräsen und dergleichen.

Vorteilhafterweise kann die Folie eine Dicke von ≤ 80 µm, vorzugsweise ≤ 60 µm und/oder von ≥ 1 µm, vorzugsweise ≥ 10 µm haben. Vorteilhafterweise kann die Folie beispielsweise eine Polyethylenfolie sein.

Der zumindest eine Kanal kann vorteilhafterweise auf eine Tiefe von ≤ 300 µm, vorzugsweise ≤ 200 µm und/oder vorzugsweise ≥ 10 µm, vorzugsweise ≥ 50 µm in dem Grundkörper des Fluidchips eingeprägt sein. Diese Tiefen sind insbesondere vorteilhaft für die Bestrahlung mit niederenergetischer Elektronenstrahlung, da bei diesen Tiefen gewährleistet ist, dass das im Kanal fließende Fluid auf seiner ganzen Tiefe der Bestrahlung ausgesetzt ist.

Die Herstellung des Fluidchips mittels in einem Grundkörper eingeprägter Kanäle, die durch eine Folie abgeschlossen werden, ist vorteilhaft, da diese Struktur kostengünstig als Einwegelement herstellbar ist.

Die Erfindung betrifft außerdem ein Bestrahlungsverfahren zum Bestrahlen eines Fluids. Es wird dabei eine modulare Bestrahlungsvorrichtung verwendet, wie sie vorstehend beschrieben wurde. Das Bestrahlungsverfahren sieht vor, dass zumindest eine der zumindest einen Trägerkassetten der modularen Bestrahlungsvorrichtung in das Hauptmodul eingesetzt wird, und danach, optional anschließend, optional unmittelbar anschließend, die Expositionsoberfläche der zumindest einen Trägerkassette mit ionisierender Strahlung bestrahlt wird, während gleichzeitig ein zu bestrahlendes Fluid durch die zumindest eine Bestrahlungsleitung bewegt wird und danach, optional anschließend, optional unmittelbar anschließend, die Trägerkassette aus dem Hauptmodul entfernt wird.

Diese Verfahrensführung ist vorteilhaft, da sie sehr effizient ist. Es ist möglich, die Trägerkassette durch Füllen der entsprechenden Pumpen mit Fluid vorzubereiten, dann in einem zeitlich kurzen Schritt in das Hauptmodul einzusetzen, die Bestrahlung und den Fluidtransport auszuführen und die Trägerkassette wieder zu entfernen. Vorteilhafterweise können eine Vielzahl von Trägerkassetten eingesetzt werden, die dann individuell vorbereitet werden können und in kurzer Folge nacheinander in das Hauptmodul eingesetzt werden können, um die Bestrahlung auszuführen. Besonders vorteilhaft können die Trägerkassetten vollständig als Einwegelemente ausgebildet sein, da sie die aufwändigen Aktoren nicht enthalten. Hierdurch können sie ohne das Erfordernis von Sterilisierung nach Entnahme der bearbeiteten Fluide entsorgt werden. Da das Fluid niemals mit dem Hauptmodul in Berührung kommt, kann eine Querkontamination zwischen Fluiden in unterschiedlichen Trägerkassetten ausgeschlossen werden. Eine große Zahl getrennter Fluidproben kann auf diese Weise effizient bestrahlt werden.

Sind mehrere Trägerkassetten vorgesehen, kann das Verfahren also so ausgeführt werden, dass einmal oder mehrmals nach dem Entfernen der jeweils vorausgehenden Trägerkassette aus dem Hauptmodul zumindest eine weitere Trägerkassette in das Hauptmodul eingesetzt wird und dann die Expositionsoberfläche der zumindest einen weiteren Trägerkassette mit ionisierender Strahlung bestrahlt wird, während ein zu bestrahlendes Fluid durch die zumindest eine Bestrahlungsleitung dieser weiteren Trägerkassette bewegt wird.

Eine besonders vorteilhafte Verfahrensführung kann vorsehen, dass ein Desinfektionsmittel durch die Bestrahlungsleitung bewegt wird, nachdem die zumindest eine Trägerkassette in das Hauptmodul eingesetzt wurde und bevor das zu bestrahlende Fluid durch die Bestrahlungsleitung bewegt wird. Auf diese Weise kann sichergestellt werden, dass das zu bestrahlende Fluid bei der Bestrahlung steril bleibt.

Eine vorteilhafte Verfahrensführung kann außerdem vorsehen, dass ein Zellmedium in und/oder durch die Bestrahlungsleitung bewegt wird, nachdem die zumindest eine Trägerkassette in das Hauptmodul eingesetzt wurde und bevor das zu bestrahlende Fluid durch die Bestrahlungsleitung bewegt wird und/oder nachdem das zu bestrahlende Fluid durch die Bestrahlungsleitung bewegt wurde. Insbesondere ist es vorteilhaft, wenn das Zellmedium nach einem eventuellen Desinfektionsmittel durch die Bestrahlungsleitung bewegt wird, da hierdurch eine Vermischung des zu bestrahlenden Fluids mit Desinfektionsmittel vermieden werden kann.

Vorteilhaft ist es, wenn der Fluidtransport durch die Bestrahlungsleitung dadurch bewirkt wird, dass die entsprechenden der Pumpen einen Unterdruck erzeugen. Das Fluid wird also jeweils durch die Bestrahlungsleitung gesaugt. Auf diese Weise kann ein unbeabsichtigtes Austreten von Fluid verhindert werden.

Das erfindungsgemäße Verfahren eignet sich besonders vorteilhaft zur Bestrahlung von Zellsuspensionen, Virussuspension, Medium, Serum und/oder Blutproben. Das zu bestrahlende Fluid kann also vorteilhaft eine Zellsuspension enthalten oder sein.

Zur Bestrahlung kommen besonders vorteilhaft ionisierende Strahlung, insbesondere Elektronen und/oder UV-Strahlung in Frage. Entsprechend kann die Bestrahlungsquelle eine Elektronenquelle oder eine UV-Strahlungsquelle sein.

Im Folgenden soll die Erfindung anhand einiger Figuren beispielhaft erläutert werden. Die in den Figuren gezeigten Merkmale können auch unabhängig von dem entsprechenden Beispiel realisiert sein und unter den verschiedenen Beispielen miteinander kombiniert werden.

Es zeigt:
- Fig. 1A, B, C:: eine modulare Bestrahlungsvorrichtung,
- Fig. 2A, B, C:: eine modulare Bestrahlungsvorrichtung,
- Fig. 3A, B, C:: eine Trägerkassette mit einigen Elementen eines Hauptmoduls,
- Fig. 4A, B, C:: eine Trägerkassette mit Elementen des Hauptmoduls,
- Fig. 5A, B:: eine Trägerkassette
- Fig. 6A, B:: einen Fluidchip,
- Fig. 7A, B, C:: eine beispielhafte schematische Vorrichtung, in der ein Verfahren zur Bestrahlung eines Fluids ausführbar ist,
- Fig. 8:: eine beispielhafte schematische Vorrichtung, in der ein Verfahren zur Bestrahlung eines Fluids ausführbar ist,
- Fig. 9:: eine beispielhafte schematische Vorrichtung, in der ein Verfahren zur Bestrahlung eines Fluids ausführbar ist, und
- Fig. 10:: eine beispielhafte schematische Vorrichtung, in der ein Verfahren zur Bestrahlung eines Fluids ausführbar ist,.

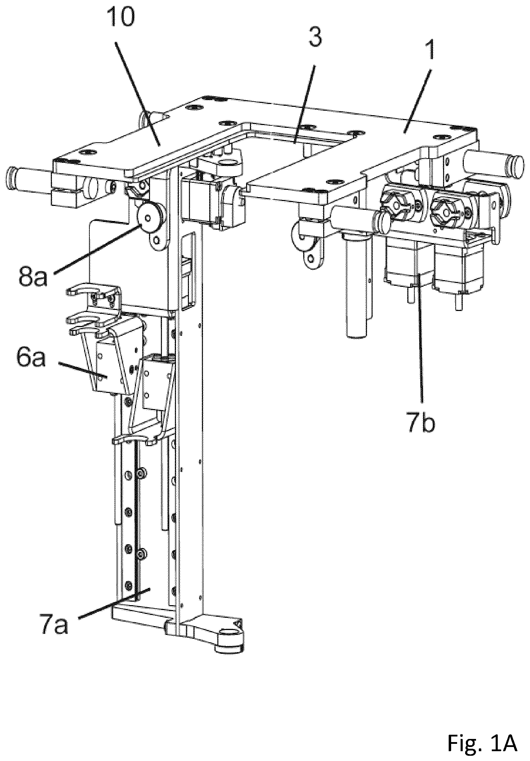

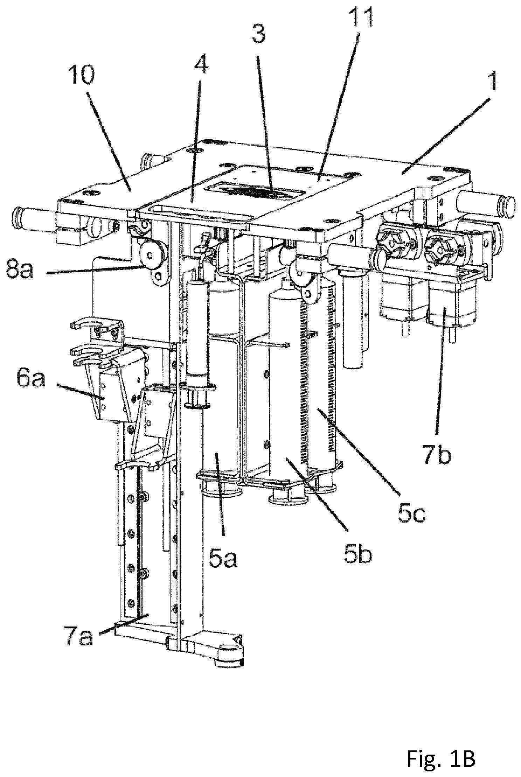

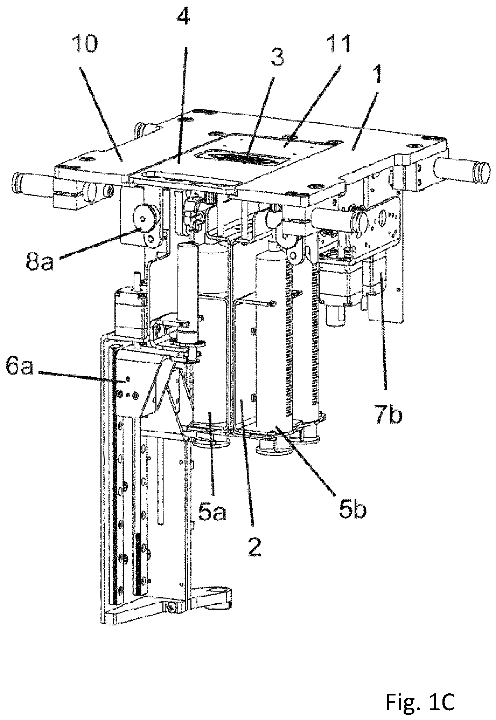

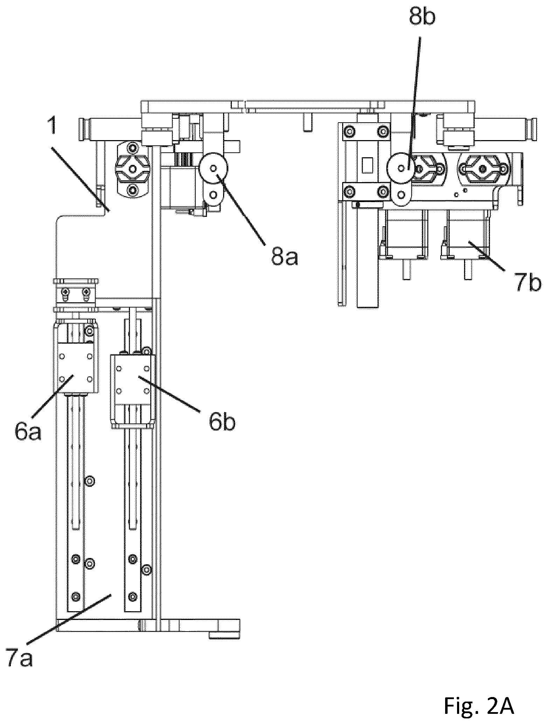

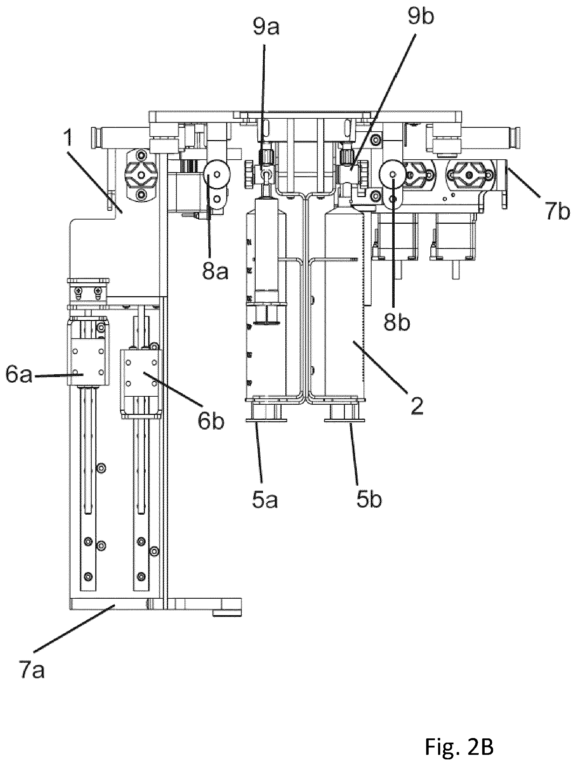

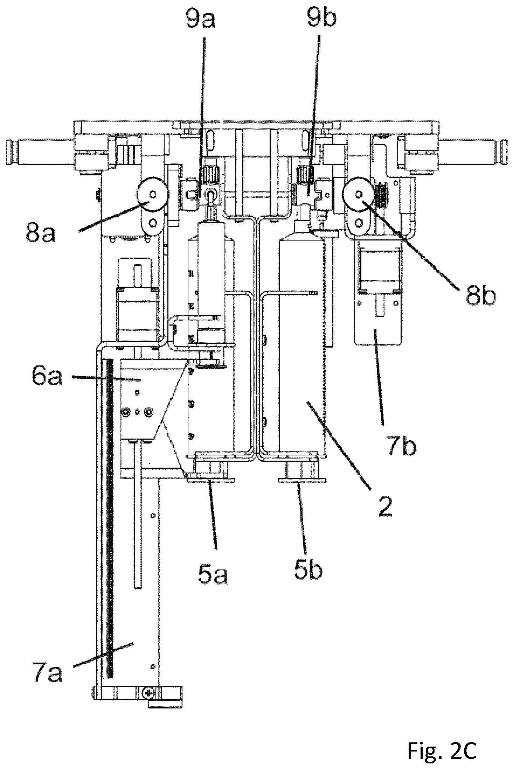

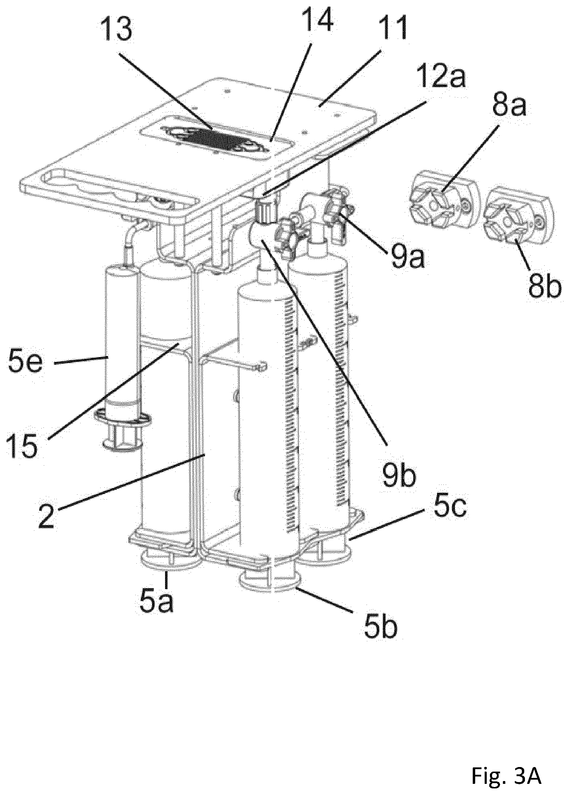

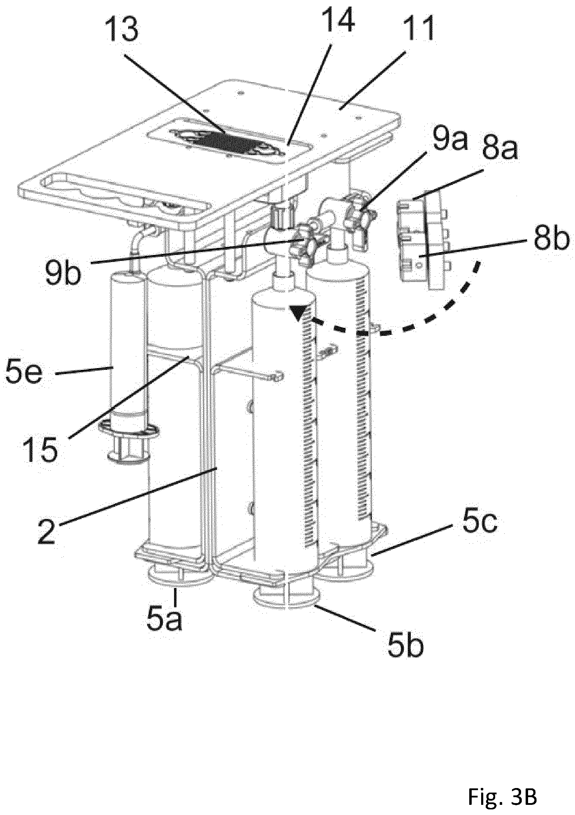

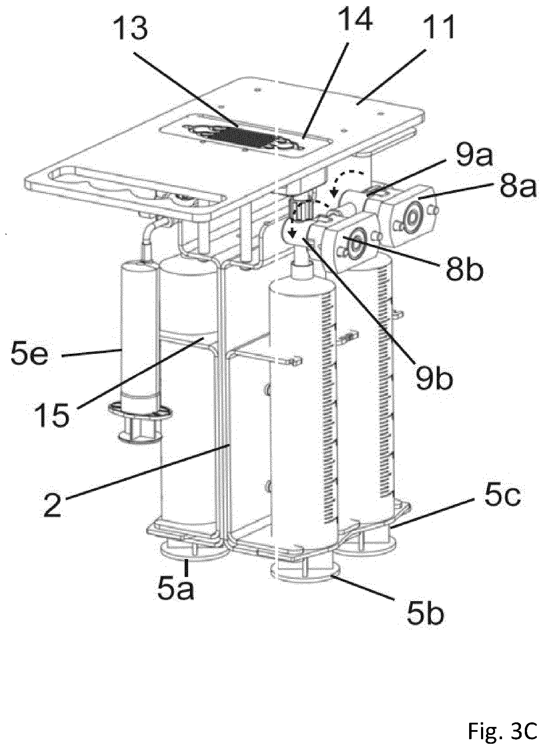

Fig. 1A, B, C zeigen eine modulare Bestrahlungsvorrichtung gemäß der Erfindung mit einem Hauptmodul 1 und einer Trägerkassette 2. In Fig. 1A ist die Trägerkassette 2 nicht im Hauptmodul angeordnet und daher nicht gezeigt. In Fig. 1B und C ist die Trägerkassette 2 in einer Aufnahme 3 des Hauptmoduls eingesetzt. Die Fig. 2A, B und C zeigen die Bestrahlungsvorrichtung der Fig. 1 jeweils in einer Seitenansicht. Die Trägerkassette 2 weist eine Expositionsoberfläche 4 auf, auf der zumindest eine Bestrahlungsleitung 13 verläuft, die in Fig. 3, 4 und 5 zu erkennen ist. Durch die Bestrahlungsleitung 13 ist ein zu bestrahlendes Fluid leitbar.

Die Trägerkassette 2 weist außerdem zumindest eine Pumpe 5a, 5b auf, die über Fluidleitungen an der Bestrahlungsleitung 13 angeschlossen ist. Das Hauptmodul 1 weist neben der Aufnahme 3, in die die Trägerkassette 2 zerstörungsfrei entfernbar einsetzbar ist, zumindest einen Pumpenaktor 6a, 6b für jede der Pumpen 5a, 5b auf, mit dem die entsprechende der Pumpen 5a, 5b aktuierbar ist, wenn die Trägerkassette 2 in die Aufnahme 3 eingesetzt ist. Im in den Fig. 1 und 2 gezeigten Beispiel sind zwei Pumpen 5a, 5b vorgesehen, die hier als Spritzen ausgestaltet sind. Entsprechend weist das Hauptmodul 2 der Aktoren 6a, 6b auf, die hier Linearaktoren sind.

Die Fig. 1A und 2A zeigen das Hauptmodul jeweils ohne eingesetzte Trägerkassette 2. Die Fig. 1B und 2B zeigen das Hauptmodul 1 mit eingesetzter Trägerkassette 2. Das Hauptmodul 1 weist in diesen Beispielen zwei klappbare Seitenteile 7a, 7b auf, welche Ventilaktoren 8a, 8b sowie die Linearaktoren 6a und 6b aufweisen. Die Trägerkassette 2 weist außerdem Ventile mit Ventilhähnen 9a, 9b auf, durch welche ein Fluidfluss zwischen den Pumpen 5a, 5b und der Bestrahlungsleitung 13 steuerbar ist. Die Linearaktoren 6a, 6b und die Ventilaktoren 8a und 8b sind am Hauptmodul 1 so angeordnet, dass sie, wenn die Seitenteile 7a, 7b nach Einsetzen der Trägerkassette 2 geschlossen werden, an den Pumpen 5a, 5b und den Ventilähnen 9a, 9b angreifen, so dass diese aktuierbar sind. Im in Fig. 1C und 2C gezeigten Beispiel wird die Pumpe 5a durch den Aktor 6a bedient, während die Pumpe 5b nicht aktuiert ist, sondern als Vorratsgefäß für Fluid dient. Der Aktor 6b aktuiert eine in den Figuren nicht gezeigte Pumpe, die hinter der Pumpe 5a angeordnet ist.

Die Pumpen 5a, 5b sind hier als Spritzen ausgestaltet mit jeweils einem Kolben, der in einem zylindrischen Zylinder verläuft. Im gezeigten Beispiel sind die Spritzen mit ihrer Kolbenbewegungsrichtung vertikal angeordnet und münden mit ihrer Auslassöffnung am oberen Ende in die Ventile 9a, 9b.

Das Hauptmodul 1 ist als Gestänge mit vier parallelen Stangen ausgestaltet, die vertikal stehen und eine Hauptmoduloberfläche 10 tragen, die das Hauptmodul nach oben abschließt. Die Trägerkassette 2 weist eine Expositionsoberfläche 11 auf, die koplanar zur Hauptmoduloberfläche 10 ist, wenn die Trägerkassette 2 in die Aufnahme 3 eingesetzt ist. Die klappbaren Seitenteile 7a, 7b sind an den parallelen Stangen des Hauptmoduls 1 angeordnet und um diese drehbar, um in den in Fig. 1C und 2C gezeigten geschlossenen Zustand geklappt zu werden.

Fig. 3A, 3B und 3C zeigen beispielhaft eine Trägerkassette 2, wie sie in den Fig. 1 und 2 gezeigt ist, im Detail. Die Trägerkassette weist hier vier Pumpen 5a, 5b, 5c, 5d auf, von denen drei zu erkennen sind. Die Pumpen sind wiederum als Spritzen ausgestaltet. Die Pumpen 5b und 5c sind über Ventile 9a und 9b und eine Fluidleitung 12 an einer Bestrahlungsleitung 13 angeschlossen, die hier als Kanalstruktur in einem Fluidchip 14 ausgestaltet ist. Die Trägerkassette weist hier eine Tragestruktur 15 auf, in die die Spritzen 5a, 5b, 5c, 5d entnehmbar eingesetzt sind. Die Zylinderachsen der Spritzen 5a, 5b, 5c, 5d liegen dabei parallel zueinander.

Die Ventile 9a, 9b sind hier Dreiwegeventile, die durch Ventilhähne verstellbar sind. Die Fig. 3A, 3B, 3C zeigen Ventilaktoren 8a, 8b, die über Kopplungselemente an den Hähnen der Ventile 9a, 9b angreifen können und diese verdrehen können. Die Aktoren 8a, 8b sind dabei Drehaktoren. Die Aktoren 8a, 8b sind nicht Teil der Trägerkassette 2, sondern Teil des Hauptmoduls 1, dessen andere Bestandteile in Fig. 3 der Übersichtlichkeit halber nicht gezeigt sind. Die Fig. 3A, 3B und 3C zeigen die Ventilaktoren 8a, 8b in unterschiedlichen Positionen relativ zu den Ventilen 9a, 9b. Diese Positionen bilden dabei das Schließen jenes der klappbaren Seitenteile 7b ab, an dem die Ventilaktoren 8a, 8b angeordnet sind. Fig. 3C zeigt dabei den geschlossenen Zustand sowie durch Pfeile angedeutet die drehende Aktuierung der Ventile 9a, 9b durch die Ventilaktoren 8a, 8b. Die Ventilaktoren 8a, 8b übertragen das Drehmoment formschlüssig auf die Ventile 9a, 9b. Im gezeigten Beispiel können die Kopplungselemente der Ventilaktoren 8a, 8b eine negative Form der Hähne der Ventile 9a, 9b aufweisen. Es sei jedoch angemerkt, dass die Ventile auch pneumatisch, hydraulisch, elektrisch, magnetisch oder auf andere Weise gestellt werden können. Auch die Verwendung von drehbaren Ventilen ist nur eine beispielhafte Option.

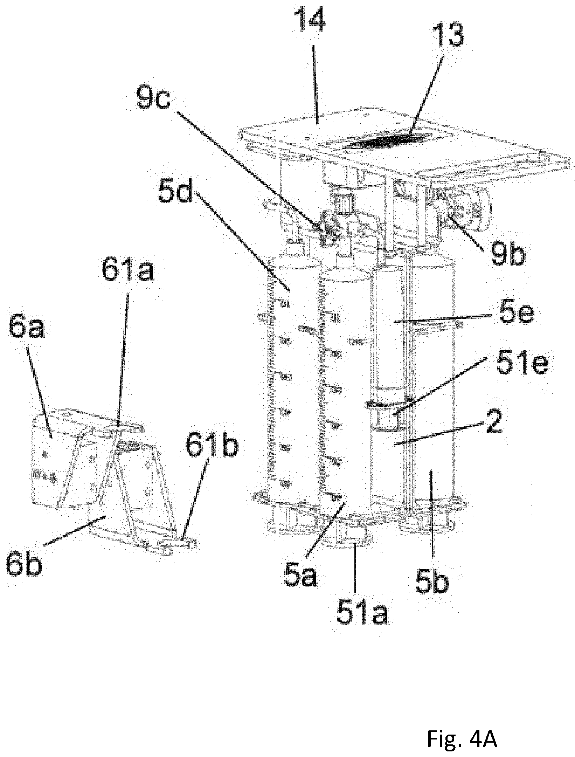

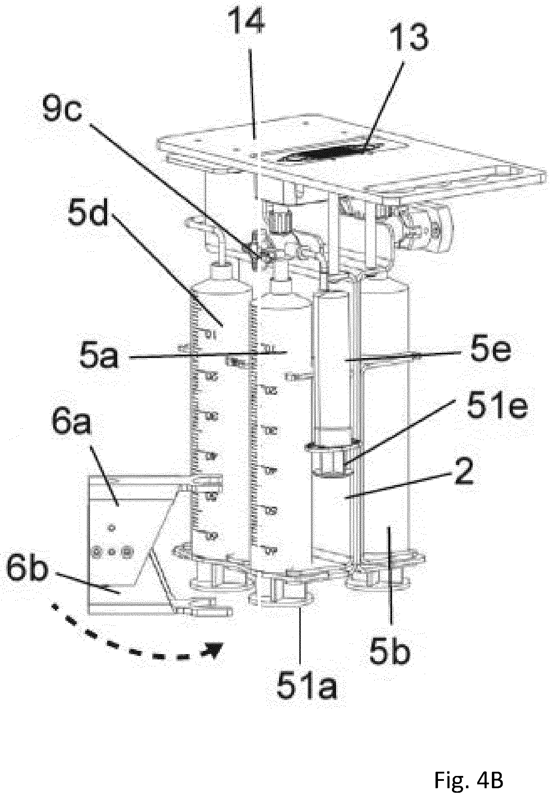

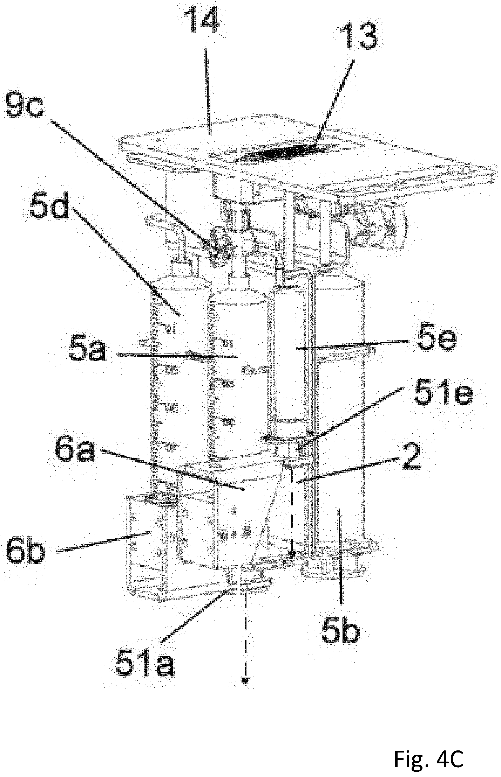

Die Fig. 4A, 4B, 4C zeigen die in Fig. 3 gezeigte Trägerkassette um 90° um eine vertikale Achse gedreht. Es kommt daher die in Fig. 3 nicht zu erkennende Pumpe 5d zum Vorschein, die ebenfalls neben den anderen Pumpen 5a, 5b, 5c zu diesen parallel angeordnet ist. Darüber hinaus ist hier ein Ventil 9c zu erkennen, über das die Pumpe 5a mit der Bestrahlungsleitung 13 verbunden ist. Soweit hier nichts Anderes gesagt wird, gilt die Beschreibung zu Fig. 3 auch für Fig. 4.

Fig. 4 zeigt zusätzlich zu der Trägerkassette 2 Elemente 6a, 6b, die Teil von Pumpenaktoren sind und daher Teil des Hauptmoduls 1 sind. Vom Hauptmodul 1 sind hier nur die an den Pumpen angreifenden Bestandteile der Ventilaktoren 6a, 6b gezeigt, während alle anderen Bestandteile des Hauptmoduls 1 der Übersichtlichkeit halber ausgeblendet sind. Die Fig. 4A, 4B und 4C zeigen die Position der Pumpenaktoren 6a, 6b relativ zur Trägerkassette 2 im Verlauf des Schließens des klappbaren Seitenteils 7a des Hauptmoduls.

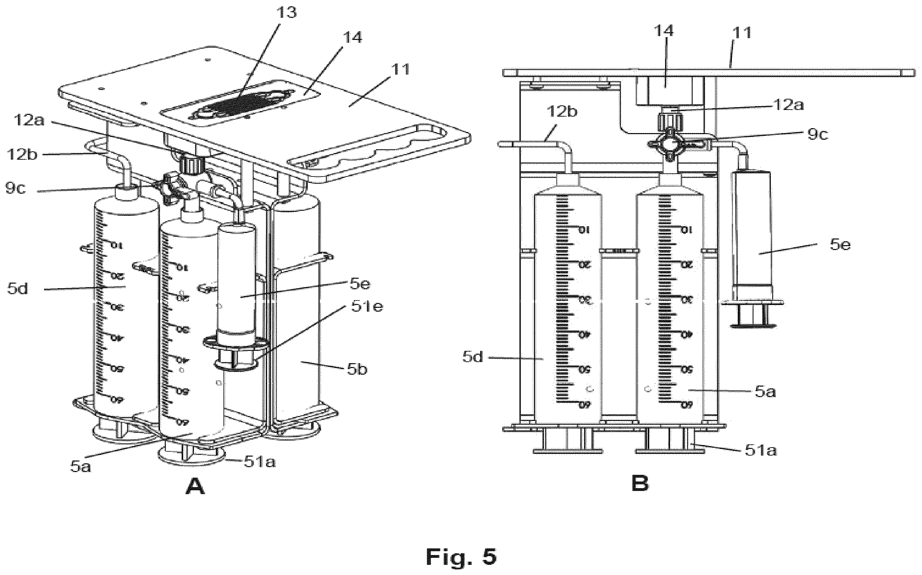

Die Pumpenaktoren 6a und 6b weisen jeweils eine Einbuchtung 61a bzw. 61b auf. Die Spritze 5a weist eine Endfläche 51a auf, die über eine Kolbenstange des Kolbens der Spritze 5a übersteht. Die kleinere Spritze 5e weist entsprechend eine Endfläche 51e auf, die über den Kolben dieser Spritze hinaussteht. Im Verlauf der Fig. 5A bis 5C wird das Seitenteil 7a geschlossen und die Aktoren 6a, 6b bewegen sich auf die Spritzen 5e und 5a zu. Im in Fig. 4C gezeigten geschlossenen Zustand greift die Öffnung 61a des Aktors 6a hinter die Endfläche 51e der Spritze 5e, so dass diese durch den Aktor 6a aufziehbar ist, wie durch den Pfeil in Fig. 4C angedeutet. Gleichzeitig greift die Einbuchtung 61b des Aktors 6b hinter die Endfläche 51a der Spritze 5a, so dass die Spritze 5a durch diesen Aktor aufziehbar ist, wie dies in Fig. 4C mit dem entsprechenden Pfeil gekennzeichnet ist.

Die Fig. 5A und 5B zeigen die in den Fig. 3 und 4 gezeigte Trägerkassette nochmals in vergrößerter Form in perspektivischer Ansicht und in Seitenansicht. Die Spritze 5a ist über das Dreiwegeventil 9c mit der Fluidleitung 12a verbunden, die ihrerseits mit einem Fluidanschluss des Fluidchips 12 verbunden ist, in den die Bestrahlungsleitung 13 eingebracht ist. Die Spritze 5d ist über eine weitere Fluidleitung 12b angeschlossen. Bezüglich des weiteren Aufbaus soll auf die Beschreibung zu Fig. 3 und 4 verwiesen werden.

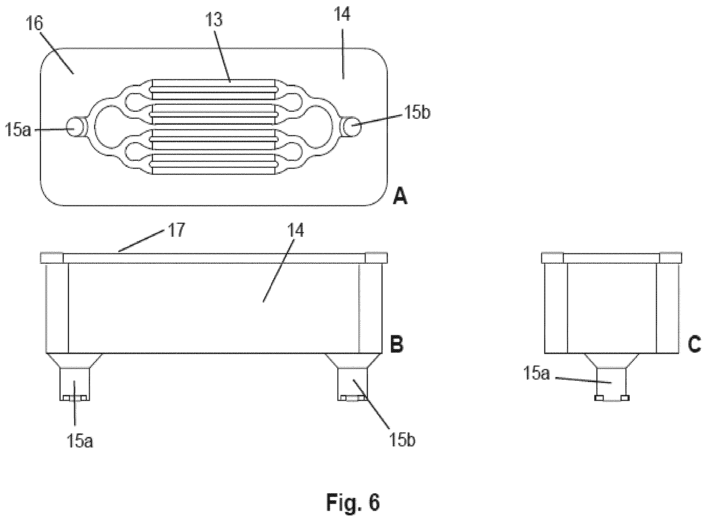

Fig. 6 zeigt beispielhaft einen Fluidchip 14, wie er in den Fig. 1 bis 5 zum Einsatz kommen kann. Der Fluidchip 14 kann dabei als monolithischer Block beispielsweise aus Polyethylen hergestellt sein, in den die Bestrahlungsleitung 13 eingeprägt oder eingeschnitten ist. Der Fluidchip 14 weist eine Grundkörperexpositionsoberfläche 16 auf, in die die Bestrahlungsleitung 13 als Kanalstruktur eingeprägt ist. Die Kanalstruktur 13 endet an Fluidanschlüssen 15a und 15b. Ausgehend vom Fluidanschluss 15a teilt sich die Kanalstruktur zunächst in zwei Kanäle, die sich jeweils wiederum in zwei Kanäle aufspalten. Diese spalten sich dann nochmals jeweils in zwei Kanäle auf und münden auf diese Weise in insgesamt acht parallele gerade Kanalabschnitte. Am gegenüberliegenden Ende vereinigen sich die geraden Kanalabschnitte wieder paarweise, bis sie in den gemeinsamen Fluidanschluss 15b münden. Im gezeigten Beispiel sind die Fluidanschlüsse 15a und 15b in zur Grundkörperexpositionsoberfläche 16 senkrechter Richtung nach unten, das heißt in Richtung von der Grundkörperexpositionsoberfläche 16 weg aus dem Fluidchip herausgeführt und können dort an die Fluidleitungen 12a, 12b angeschlossen werden. Die Grundkörperexpositionsoberfläche 16 ist im gezeigten Beispiel mit einer Folie 17 bedeckt, welche die Kanalstruktur 13 gegen Austreten von Fluid auf die Grundkörperexpositionsoberfläche 16 abschließt.

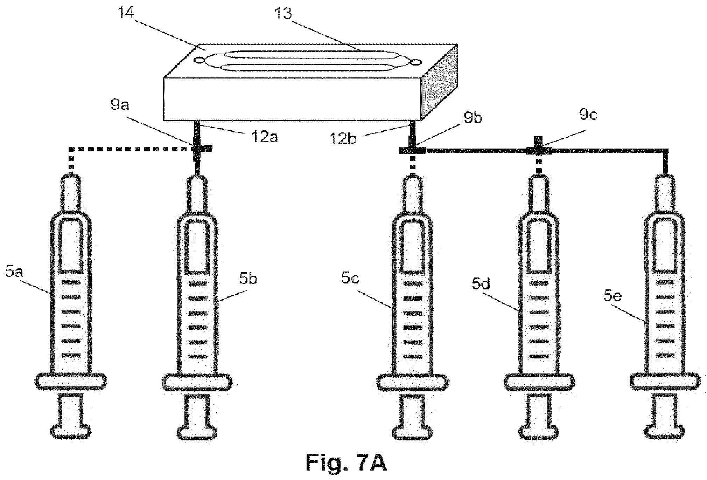

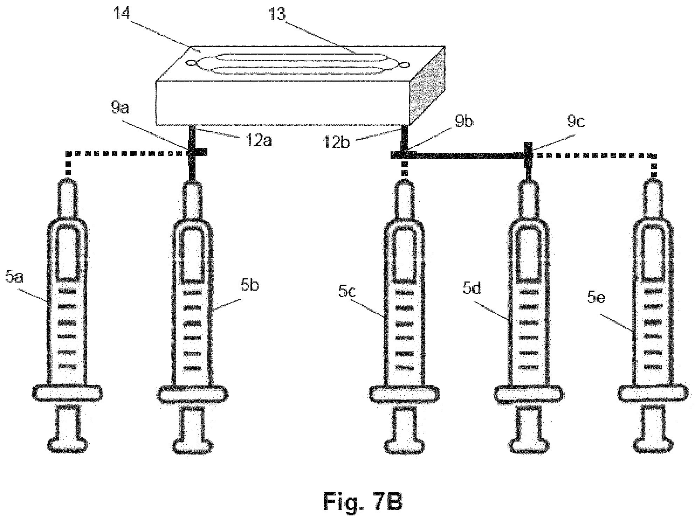

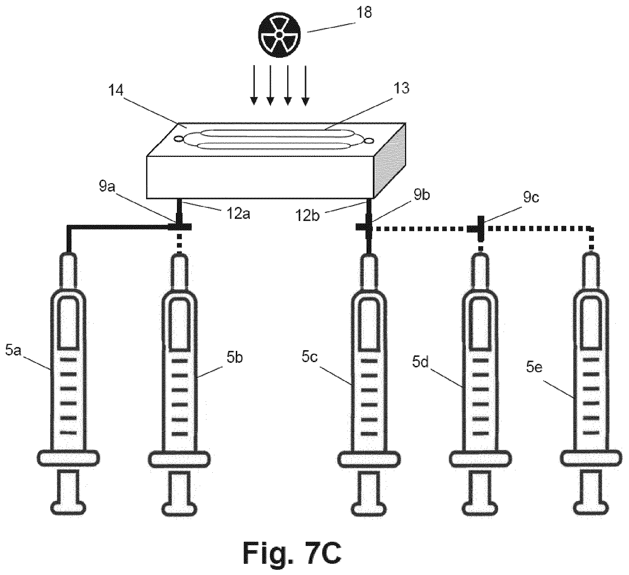

Die Fig. 7A, 7B, 7C und 7D zeigen beispielhaft, wie ein erfindungsgemäßes Verfahren in der modularen Bestrahlungsvorrichtung der Erfindung ausführbar ist. Die Fig. 7A, B, C, D zeigen dabei der Übersichtlichkeit halber nur die Spritzen 5a, 5b, 5c, 5d, 5e, die Ventile 9a, 9b, 9c und den Fluidchip 14 mit der Bestrahlungsleitung 13. Diese Elemente können wie in den Fig. 1 bis 6 gezeigt ausgestaltet sein. Die Anordnung der Elemente in den Fig. 7 ist hier nur schematisch funktional zu verstehen.

Die Ventile 9a, 9b, 9c sind hier Dreiwegeventile, mit denen schaltbar ist, welche der Spritzen 5a bis 5e mit dem Fluidchip 14 fluidleitend verbunden ist. Dabei sind die Spritzen 5a und 5b über das Dreiwegeventil 9a über eine erste Fluidleitung 12a mit einem ersten Fluidanschluss des Fluidchips 14 verbunden und die Spritzen 5c, 5d, 5e über Dreiwegeventile 9b und 9c mittels einer zweiten Fluidleitung 12b an einem zweiten Fluidanschluss des Fluidchips 14 angeschlossen.

Im Folgenden soll angenommen werden, dass die Spritze 5a der Aufnahme des bestrahlten Fluids dient, die Spritze 5b zur Aufnahme von Abfallfluid dient, die Spritze 5c eine Zellsuspension enthält, die Spritze 5d ein Zellmedium enthält und die Spritze 5e ein Desinfektionsmittel wie beispielsweise Ethanol enthält.

Nach der Herstellung und Versiegelung des Mikrofluidchips 14 können sich eventuell Keime und Ähnliches in den Bestrahlungsleitungen 13 befinden. Der Fluidchip 14 sollte daher vorteilhafterweise desinfiziert werden. Hierzu wird, wie in Fig. 7A gezeigt, das Dreiwegeventil 9a so geschaltet, dass es eine Verbindung zwischen der Abfallspritze 5b und dem Fluidchip 14 herstellt. Außerdem werden die Dreiwegeventile 9b und 9c so gestaltet, dass sie die Spritzen 5c und 5d verschließen und eine fluidleitende Verbindung zwischen der Spritze 5e und dem Fluidchip 14 herstellen. Es wird nun die Spritze 5b aufgezogen, wodurch Fluid, hier das Desinfektionsmittel, aus der Spritze 5e gesaugt und durch den Fluidchip 14 geleitet wird.

Im in Fig. 7B gezeigten nächsten Schritt bleibt die Stellung des Dreiwegeventils 9a unverändert, so dass weiterhin die Abfallspritze 5b mit dem Fluidchip 14 verbunden ist. Das Dreiwegeventil 9c, über das die Spritze 5d mit dem Fluidchip verbunden ist, wird so gestellt, dass die Spritze 5d fluidleitend mit dem Fluidchip 14 verbunden ist. Die Stellung des Dreiwegeventils 9b an der Spritze 5c bleibt unverändert. Es wird nun die Abfallspritze 5b weiter aufgezogen und dadurch Fluid, hier Zellmedium, aus der Spritze 5d in den Fluidchip 14 und durch diesen hindurch gesaugt.

Im in Fig. 7C gezeigten nächsten Schritt wird nun der Ventilhahn 9a an der ersten Fluidleitung 12a so gestellt, dass die Spritze 5a fluidleitend mit der ersten Fluidleitung 12a und damit mit der Bestrahlungsleitung 13 im Fluidchip 14 verbunden ist. Außerdem wird das Dreiwegeventil 9b so gestellt, dass die Spritzen 5d und 5e von der zweiten Fluidleitung 12b abgeschnitten sind und die Spritze 5c mit der Zellsuspension fluidleitend mit der Bestrahlungsleitung 13 verbunden ist. Außerdem wird eine Bestrahlungsquelle 18 aktiviert, die auf die Expositionsoberfläche und die Bestrahlungsleitung 13 strahlt. Es wird währenddessen dann die Spritze 5a aufgezogen und dadurch Zellsuspension aus der Spritze 5c durch den Fluidchip 14 gesaugt und in die Spritze 5a aufgenommen. Nach Abschluss der Bestrahlung kann optional nochmals das Dreiwegeventil 9b so gestaltet werden, dass nochmals die Spritze 5d mit der Bestrahlungsleitung 13 verbunden wird. Auf diese Weise kann durch weiteres Aufziehen der Spritze 5a die Kanalstruktur 13 mit Zellmedium nachgespült werden, um möglichst viele der bestrahlten Zellen aus dem Fluidchip 14 in die Spritze 5a zu spülen.

Bei Bedarf kann im Anschluss der Fluidchip 14 nochmals mit Ethanol aus der Spritze 5e nachgespült werden. Die Konstellation entspricht dabei wiederum der in Fig. 7A gezeigten. Es wird wiederum die Spritze 5b aufgezogen und dadurch Desinfektionsmittel aus der Spritze 5e durch den Fluidchip 14 gesaugt.

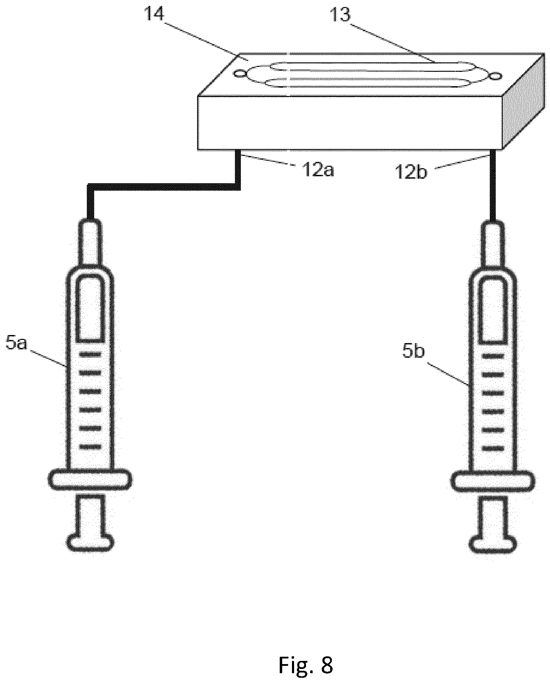

Fig. 8 zeigt beispielhaft eine sehr einfache Ausgestaltung der Erfindung, bei der lediglich eine Spritze 5a für die bestrahlte Probe sowie eine Spritze 5b für Zellsuspension vorgesehen ist. Die Spritze 5a ist über eine erste Fluidleitung 12a an einem Fluidanschluss des Fluidchips 14 angeschlossen und die Spritze 5b über eine Fluidleitung 12b an einem zweiten Fluidanschluss des Fluidchips 14. In dieser Ausgestaltung ist es nicht erforderlich, dass die Bestrahlungsvorrichtung Ventile aufweist. Es wird zur Bestrahlung lediglich die Bestrahlungsquelle 18 (hier nicht gezeigt) aktiviert und dann die Spritze 5a aufgezogen und dadurch Zellsuspension aus der Spritze 5b durch die Kanalstruktur 13 in die Spritze 5a transportiert.

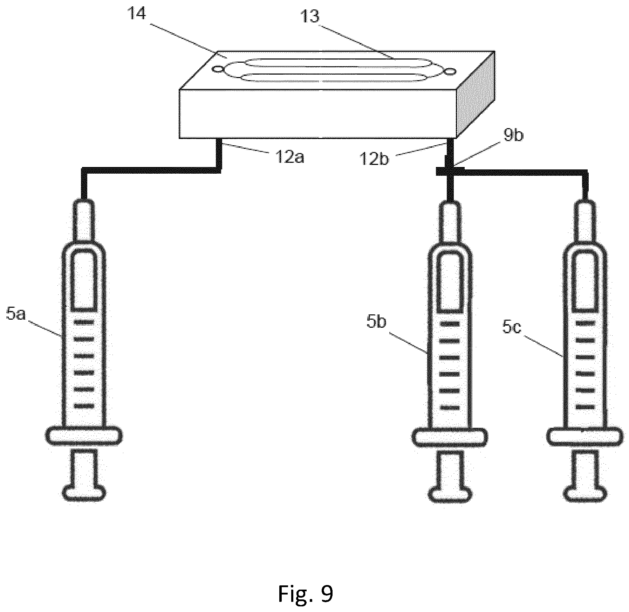

Fig. 9 zeigt eine weitere einfache Ausgestaltung der Erfindung, wobei wiederum an dem ersten Fluidanschluss des Fluidchips 14 über die Fluidleitung 12a nur eine Spritze 5a für die bestrahlte Probe angeordnet ist. Am zweiten Fluidanschluss ist über die zweite Fluidleitung 12b und ein Dreiwegeventil 9b zum einen eine Spritze 5b mit Zellsuspension und zum anderen eine Spritze 5c mit Zellmedium angeschlossen. Analog wie in den Fig. 7 gezeigt kann zunächst durch Aufziehen der Spritze 5a Zellsuspension aus der Spritze 5b durch den Fluidchip 14 gesaugt werden. Hierzu ist das Dreiwegeventil so geschaltet, dass es eine Verbindung zwischen der Spritze 5b und dem Fluidanschluss des Fluidchips 14 herstellt. Im Anschluss an die Bestrahlung kann dann das Dreiwegeventil 9b so geschaltet werden, dass es eine Verbindung zwischen der Spritze 5c mit Zellmedium und dem Fluidchip 14 herstellt und gleichzeitig die Spritze 5b schließt. Wird dann die Spritze 5a weiter aufgezogen, so wird die Kanalstruktur 13 des Fluidchips 14 mit Zellmedium nachgespült und auf diese Weise möglichst viele Zellen aus der Kanalstruktur 13 entfernt.

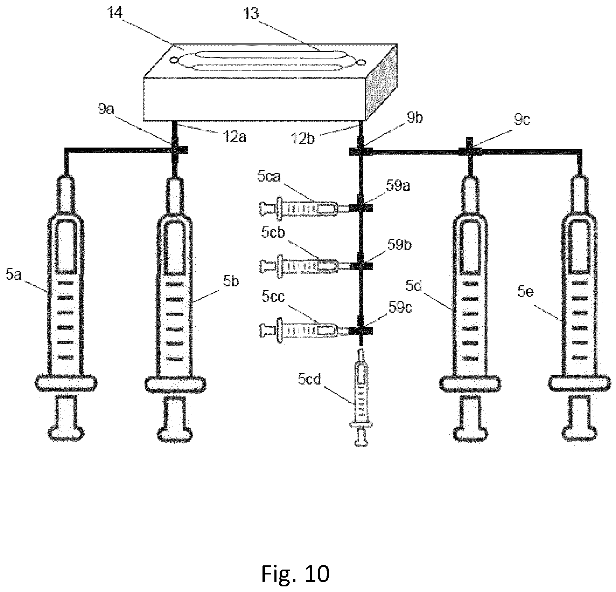

Fig. 10 zeigt eine Variante der in Fig. 7 gezeigten Situation. Fig. 10 unterscheidet sich von den Fig. 7 dadurch, dass anstelle der Spritze 5d in den Fig. 7 an dem entsprechenden Ventil 9b eine Anordnung von vier Spritzen 5ca, 5cb, 5cc und 5cd angeordnet ist, die über Dreiwegeventile 59a, 59b, 59c mit dem Ventil 9b verbunden sind. In den Spritzen 5ca, 5cb, 5cc können verschiedene Suspensionen bereitgestellt werden, die durch die Kanalstruktur 13 geleitet werden können. Mit einer Spritze 5cd kann außerdem zusätzlich Zellsuspension bereitgestellt werden, mit der das am Ventilhahn 9b angeschlossene System nachspülbar ist. Durch Stellen der Ventile 59a, 59b, 59c können die Spritzen 5ca, 5cb, 5cd und 5cd selektiv mit dem Ventil 9b verbunden werden. Das Verfahren kann dann analog zu dem in Fig. 7 gezeigten ausgeführt werden.

Die Erfindung erlaubt eine sichere und sterile Bestrahlung von Fluiden. So kann zum Beispiel der Mikrofluidchip 14 als Spritzgussteil aus Polyethylen hergestellt werden und anschließend mit einer PET/PE-Folie versiegelt werden. Das Versiegeln mit einer dünnen Folie (zum Beispiel < 60 µm) trägt dazu bei, dass nur ein geringer Anteil der Strahlung von der Folie absorbiert wird. Alle Komponenten, die mit der Zellsuspension in Berührung kommen, können vorteilhaft als Einwegteile ausgelegt sein, insbesondere der Fluidchip 14 und die Spritzen 5. Durch das modulare Konzept mit Hauptmodul und Trägerkassette ist es möglich, mehrere Trägerkassetten herzustellen und parallel zu bestücken. Auf diese Weise wird die Prozessvorbereitung parallelisierbar. Es ist möglich, das System automatisch zu entlüften, um mögliche Elastizitäten und damit verbundene Strömungsveränderungen zu vermeiden. Die Wege zwischen den Pumpen und der Bestrahlungsleitung werden vorzugsweise kurz gehalten, damit keine zusätzlichen Elastizitäten, zum Beispiel durch Silikonschläuche, entstehen, was zu Fließgeschwindigkeitsänderungen führen könnte. Außerdem kann dadurch das Totvolumen gering gehalten werden, was ein großer Vorteil bei der Herstellung von personalisierter Medizin ist. Die Schichtdicke und die Fließgeschwindigkeit des Fluids in der Bestrahlungsleitung kann präzise eingestellt und kontrolliert werden.

## Patentansprüche

1. Modulare Bestrahlungsvorrichtung, aufweisend ein Hauptmodul (1) und
zumindest eine Trägerkassette (2),
wobei die Trägerkassette (2) folgendes aufweist:
eine Expositionsoberfläche (4), auf der zumindest eine Bestrahlungsleitung (13) verläuft, wobei in der zumindest einen Bestrahlungsleitung (13) ein zu bestrahlendes Fluid leitbar ist,
sowie zumindest eine Pumpe (5a, 5b, 5c, 5d, 5e), die über eine erste Fluidleitung an der zumindest einen Bestrahlungsleitung (13) angeschlossen ist, und
wobei das Hauptmodul (1) folgendes aufweist:
zumindest eine Aufnahme (3), in welche die zumindest eine Trägerkassette (2) zerstörungsfrei entfernbar einsetzbar ist, sowie zumindest einen Pumpenaktor (6a, 6b), der so angeordnet ist, dass mit ihm die zumindest eine Pumpe (5a, 5b, 5c, 5d, 5e) aktuierbar ist, wenn die zumindest eine Trägerkassette (2) in der entsprechenden Aufnahme (3) eingesetzt ist.

2. Modulare Bestrahlungsvorrichtung nach dem vorhergehenden Anspruch, wobei die Trägerkassette (2) zumindest eine über eine zweite Fluidleitung an der Bestrahlungsleitung (13) angeschlossene weitere der Pumpen (5a, 5b, 5c, 5d, 5e) aufweist, und/oder wobei der Pumpenaktor (6a, 6b) ein Pumpenkopplungselement aufweist, das so angeordnet ist, dass es mit einem beweglichen Element der entsprechenden zumindest einen Pumpe (5a, 5b, 5c, 5d, 5e) formschlüssig in Eingriff kommt, wenn die Kassette (2) in das Hauptmodul (1) eingesetzt wird.

3. Modulare Bestrahlungsvorrichtung nach einem der vorhergehenden Ansprüche, wobei
die zumindest eine Trägerkassette (2) zumindest ein in zumindest einer der Fluidleitungen (12a, 12b) angeordnetes Ventil (9a, 9b, 9c) aufweist, mit dem ein Fluidfluss zwischen der entsprechenden Pumpe (5a, 5b, 5c, 5d, 5e), die über diese Fluidleitung (12a, 12b) an der Bestrahlungsleitung (13) angeschlossen ist, und der Bestrahlungsleitung (13) steuerbar ist, und
wobei das Hauptmodul (1) für eines, mehrere oder alle der zumindest einen Ventile (9a, 9b, 9c) jeweils einen Ventilaktor (8a, 8b) aufweist, der oder die jeweils so angeordnet sind, dass durch sie das entsprechende Ventil (9a, 9b, 9c) verstellbar ist, wenn die zumindest eine Trägerkassette (2) in der entsprechenden Aufnahme (3) angeordnet ist.

4. Modulare Bestrahlungsvorrichtung nach dem vorhergehenden Anspruch, wobei das zumindest eine Ventil (9a, 9b, 9c) einen Hahn aufweist, mit dem es zur Steuerung des Fluidflusses verstellbar ist, wobei der entsprechende Ventilaktor (8a, 8b) ein Kopplungselement aufweist, das mit dem Hahn so koppelbar ist, dass mit dem Ventilaktor (8a, 8b) eine das Ventil (9a, 9b, 9c) verstellende Kraft oder ein das Ventil (9a, 9b, 9c) verstellendes Drehmoment auf den Hahn ausübbar ist, und
wobei das Kopplungselement so angeordnet ist, dass es mit dem Hahn in Eingriff kommt, wenn die Trägerkassette (2) in das Hauptmodul (1) eingesetzt wird, und/oder wobei das zumindest eine Ventil (9a, 9b, 9c) ein Dreiwegeventil mit drei Anschlüssen aufweist oder ist, wobei an einem ersten der Anschlüsse eine der Fluidleitungen (12a, 12b) angeschlossen ist, an einem zweiten der Anschlüsse die zumindest eine Pumpe (5a, 5b, 5c, 5d, 5e) angeschlossen ist und am dritten der Anschlüsse eine weitere der Pumpen (5a, 5b, 5c, 5d, 5e) angeschlossen ist.

5. Modulare Bestrahlungsvorrichtung nach einem der vorhergehenden Ansprüche, wobei die zumindest eine Pumpe (5a, 5b, 5c, 5d, 5e) eine Fluidkammer und einen Kolben aufweist, wobei der Kolben die Fluidkammer fluiddicht abschließt und in der Fluidkammer verschiebbar ist, wobei der zumindest eine Pumpenaktor (6a, 6b) am Kolben der Pumpe (5a, 5b, 5c, 5d, 5e) angreift wenn die zumindest eine Trägerkassette (2) in die entsprechenden Aufnahme (3) eingesetzt ist und mit dem Pumpenaktor (6a, 6b) eine Kraft in einer Verschiebungsrichtung des Kolbens bewirkbar ist.

6. Modulare Bestrahlungsvorrichtung nach einem der vorhergehenden Ansprüche, wobei die zumindest eine Pumpe (5a, 5b, 5c, 5d, 5e) eine Spritze, vorzugsweise eine austauschbare Plastikspritze ist, und/oder wobei die Trägerkassette (2) drei an der zweiten Fluidleitung (12a, 12b) angeschlossene Pumpen (5a, 5b, 5c, 5d, 5e) und zwei an der ersten Fluidleitung (12a, 12b) angeschlossene Pumpen (5a, 5b, 5c, 5d, 5e) aufweist.

7. Modulare Bestrahlungsvorrichtung nach einem der vorhergehenden Ansprüche,
wobei das Hauptmodul (1) eine Hauptmoduloberfläche (10) aufweist, und wobei die Expositionsoberfläche (11) der Trägerkassette (2) und die Hauptmoduloberfläche (10) koplanar sind, wenn die Trägerkassette (2) in der entsprechenden Aufnahme (3) des Hauptmoduls (1) eingesetzt ist, und/oder wobei
das Hauptmodul (1) genau für jede an einer der Fluidleitungen (12a, 12b), die vorzugsweise als Ableitung für Fluid verwendbar ist, angeschlossene Pumpe (5a, 5b, 5c, 5d, 5e) jeweils einen Aktor aufweist, mit dem die entsprechende Pumpe (5a, 5b, 5c, 5d, 5e) aktuierbar ist.

8. Modulare Bestrahlungsvorrichtung nach einem der Ansprüche 2 bis 7, wobei
das Hauptmodul (1) zwei bewegliche und/oder klappbare Seitenteile (7a, 7b) aufweist,
wobei vorzugsweise solche der Aktoren, mit denen die über die erste Fluidleitung (12a, 12b) an der Bestrahlungsleitung (13) angeschlossenen Pumpen (5a, 5b, 5c, 5d, 5e) bewegbar sind, an einem der Seitenteile (7a, 7b) angeordnet sind, und wobei solche der Aktoren, mit denen über die zweite Fluidleitung (12a, 1b) an der Bestrahlungsleitung (13) angeschlossenen Pumpen (5a, 5b, 5c, 5d, 5e) bewegbar sind, am anderen der Seitenteile (7a, 7b) angeordnet sind.

9. Modulare Bestrahlungsvorrichtung nach einem der vorhergehenden Ansprüche, wobei
die Trägerkassette (2) einen Fluidchip (14) aufweist,
wobei der Fluidchip (14) einen Grundkörper aufweist,
wobei der Grundkörper eine Grundkörperexpositionsoberfläche (16) aufweist, in der die zumindest eine Bestrahlungsleitung (13) ausgebildet ist, wobei die zumindest eine Bestrahlungsleitung (13)eine Kanalstruktur mit zumindest einem Kanal ist,
wobei der Grundkörper weiter einen ersten Fluidanschluss (15a), an den die erste Fluidleitung (12a) angeschlossen ist, und einen zweiten Fluidanschluss (15b), an den die zweite Fluidleitung (12b) angeschlossen ist, aufweist,
wobei der Grundköper außerdem eine Folie (17), welche auf der Grundkörperexpositionsoberfläche (16) angeordnet ist und die Kanalstruktur bedeckt, aufweist,
wobei die Folie (17) die Kanalstruktur gegen Austreten von Fluid auf die Grundkörperexpositionsoberfläche (16) abdichtet.

10. Modulare Bestrahlungsvorrichtung nach dem vorhergehenden Anspruch, wobei die Kanalstruktur eine Mehrzahl der Kanäle aufweist, die an ihren jeweiligen Enden in den jeweiligen Fluidanschluss (15a, 15b) zusammenlaufen, wobei vorzugsweise die Kanäle an ihren Enden jeweils paarweise in gemeinsame Kanäle zusammenlaufen und die gemeinsamen Kanäle ihrerseits so oft jeweils paarweise in gemeinsame Kanäle zusammenlaufen bis genau zwei gemeinsame Kanäle in einen der Fluidanschlüsse (15a, 15b) zusammenlaufen.

11. Modulare Bestrahlungsvorrichtung nach einem der Ansprüche 9 bis 10, wobei der Grundkörper ein monolithischer Block, vorzugsweise ein Spritzgussteil, ist, in den die Kanalstruktur eingeprägt und/oder eingeschnitten ist, und/oder wobei die Folie eine Dicke von kleiner oder gleich 80µm, vorzugsweise von kleiner oder gleich 60 µm und/oder von größer oder gleich als 1µm, vorzugsweise größer oder gleich 10 µm hat und/oder eine Polyethylenfolie ist, und/oder wobei eine Tiefe des zumindest einen Kanals kleiner oder gleich 300 µm, vorzugsweise kleiner oder gleich 200 µm und/oder vorzugsweise größer oder gleich 10 µm, vorzugsweise größer oder gleich 50 µm beträgt.

12. Bestrahlungsverfahren zum Bestrahlen eines Fluides, wobei in einer modularen Bestrahlungsvorrichtung nach einem der vorhergehenden Ansprüche
zumindest eine der zumindest einen Trägerkassetten (2) in das Hauptmodul (1) eingesetzt wird,
danach die Expositionsoberfläche (4) der zumindest einen Trägerkassette (2) mit ionisierender Strahlung bestrahlt wird, während ein zu bestrahlendes Fluid durch die zumindest eine Bestrahlungsleitung (13) bewegt wird, und danach die Trägerkassette (2) aus dem Hauptmodul (1) entfernt wird.

13. Bestrahlungsverfahren nach dem vorhergehenden Anspruch, wobei einmal oder mehrmals nach dem Entfernen der jeweils vorausgehenden Trägerkassette (2) aus dem Hauptmodul (1) zumindest eine weitere Trägerkassette in das Hauptmodul (1) eingesetzt wird und dann die Expositionsoberfläche (4) der zumindest einen weiteren Trägerkassette (2) mit ionisierender Strahlung bestrahlt wird, während ein zu bestrahlendes Fluid durch die zumindest eine Bestrahlungsleitung (13) dieser Trägerkassette (2) bewegt wird.

14. Bestrahlungsverfahren nach einem der beiden vorhergehenden Ansprüche,
wobei ein Desinfektionsmittel durch die Kanalstruktur des Fluidchips (14) bewegt wird, nachdem die zumindest eine Trägerkassette (2) in das Hauptmodul (1) eingesetzt wurde und bevor das zu bestrahlende Fluid durch die Bestrahlungsleitung (13) bewegt wird, und/oder wobei ein Zellmedium in die Kanalstruktur bewegt wird, nachdem die zumindest eine Trägerkassette (2) in das Hauptmodul (1) eingesetzt wurde und bevor das zu bestrahlende Fluid durch die Bestrahlungsleitung (13) bewegt wird und/oder nach dem das zu bestrahlende Fluid durch die Kanalstruktur bewegt wurde.

15. Bestrahlungsverfahren nach einem der Ansprüche 12 bis 14 wobei der Fluidtransport durch die Bestrahlungsleitung (13) dadurch bewirkt wird, dass die entsprechende der Pumpen (5a, 5b, 5c, 5d, 5e) einen Unterdruck erzeugt, und/oder wobei das zu bestrahlende Fluid eine Zellsuspension, Virussuspension, Medium, Serum und/oder Blutprobe enthält oder ist, und/oder wobei die ionisierende Strahlung Elektronen oder UV-Strahlung ist.

## Claims

1. A modular irradiation device, comprising:
a main module (1) and
at least one carrier cassette (2),
the carrier cassette (2) comprising the following:
an exposure surface (4), on which the at least one irradiation line (13) runs, a fluid to be irradiated being conductible in the at least one irradiation line (13),
and at least one pump (5a, 5b, 5c, 5d, 5e), which is connected via a first fluid line to the at least one irradiation line (13), and
the main module (1) comprising the following:
at least one receptacle (3) in which the at least one carrier cassette (2) can be inserted so as to be removable without destruction, and
at least one pump actuator (6a, 6b), which is arranged so as to be able to actuate the at least one pump (5a, 5b, 5c, 5d, 5e) thereby when the at least one carrier cassette (2) is inserted into the corresponding receptacle (3).

2. The modular irradiation device according to the preceding claim,
wherein the carrier cassette (2) comprises at least one further of the pumps (5a, 5b, 5c, 5d, 5e), which is connected via a second fluid line to the irradiation line (13), and/orwherein the pump actuator (6a, 6b) comprises a pump coupling element, which is arranged so as to become engaged with a movable element of the corresponding at least one pump (5a, 5b, 5c, 5d, 5e) in a form-locked manner when the cassette (2) is being inserted into the main module (1).

3. The modular irradiation device according to any one of the preceding claims, wherein
the at least one carrier cassette (2) comprises at least one valve (9a, 9b, 9c), which is arranged in at least one of the fluid lines (12a, 12b) and by way of which a fluid flow between the corresponding pump (5a, 5b, 5c, 5d, 5e), which is connected via this fluid line (12a, 12b) to the irradiation line (13), and the irradiation line (13) can be controlled, and
the main module (1) comprises a respective valve actuator for one, several or all of the at least one valves (9a, 9b, 9c), the valve actuators (8a, 8b) being in each case arranged so as to be able to adjust the corresponding valve (9a, 9b, 9c) when the at least one carrier cassette (2) is arranged in the corresponding receptacle (3).

4. The modular irradiation device according to the preceding claim, wherein the at least one valve (9a, 9b, 9c) comprises a stopcock by way of which the valve can be adjusted for controlling the flow of fluid,
the corresponding valve actuator (8a, 8b) comprising a coupling element, which can be coupled to the stopcock in such a way that a force adjusting the valve (9a, 9b, 9c) or a torque adjusting the valve (9a, 9b, 9c) can be exerted on the stopcock by way of the valve actuator (8a, 8b), and
the coupling element being arranged so as to become engaged with the stopcock when the carrier cassette (2) is being inserted into the main module (1), and/or wherein the at least one valve (9a, 9b, 9c) comprises or is a three-way valve having three ports, one of the fluid lines (12a, 12b) being connected to a first of the ports, the at least one pump (5a, 5b, 5c, 5d, 5e) being connected to a second of the ports, and a further of the pumps (5a, 5b, 5c, 5d, 5e) being connected to the third of the ports.

5. The modular irradiation device according to any one of the preceding claims, wherein the at least one pump (5a, 5b, 5c, 5d, 5e) comprises a fluid chamber and a plunger, the plunger sealing the fluid chamber in a fluid-tight manner and being displaceable in the fluid chamber, the at least one pump actuator (6a, 6b) engaging on the plunger of the pump (5a, 5b, 5c, 5d, 5e) when the at least one carrier cassette (2) is inserted into the corresponding receptacle (3), and a force being effectuatable in a displacement direction of the plunger by way of the pump actuator (6a, 6b).

6. The modular irradiation device according to any one of the preceding claims, wherein the at least one pump (5a, 5b, 5c, 5d, 5e) is a syringe, preferably an exchangeable plastic syringe,wherein the carrier cassette (2) comprises three pumps (5a, 5b, 5c, 5d, 5e) that are connected to the second fluid line (12a, 12b), and two pumps (5a, 5b, 5c, 5d, 5e) that are connected to the first fluid line (12a, 12b).

7. The modular irradiation device according to any one of the preceding claims,
wherein the main module (1) includes a main module surface (10), and the exposure surface (11) of the carrier cassette (2) and the main module surface (10) are coplanar when the carrier cassette (2) is inserted into the corresponding receptacle (3) of the main module (1), and/or wherein
the main module (1) comprises exactly one actuator for each pump (5a, 5b, 5c, 5d, 5e) that is connected to one of the fluid lines (12a, 12b), which can preferably be used as a discharge line for fluid, the corresponding pump (5a, 5b, 5c, 5d, 5e) being actuatable by way of the actuator.

8. The modular irradiation device according to any one of claims 2 to 7, wherein
the main module (1) comprises two movable and/or foldable side parts (7a, 7b),
preferably those actuators by way of which the pumps (5a, 5b, 5c, 5d, 5e) that are connected via the first fluid line (12a, 12b) to the irradiation line (13) can be actuated being arranged at one of the side parts (7a, 7b), and those actuators by way of which the pumps (5a, 5b, 5c, 5d, 5e) that are connected via the second fluid line (12a, 1b) to the irradiation line (13) can be actuated being arranged at the other of the side parts (7a, 7b).

9. The modular irradiation device according to any one of the preceding claims, wherein
the carrier cassette (2) comprises a fluid chip (14),
the fluid chip (14) comprising a base body,
the base body including a base body exposure surface (16) in which the at least one irradiation line (13) is formed, the at least one irradiation line (13) being a channel structure including at least one channel,
the base body furthermore comprising a first fluid connection (15a) to which the first fluid line (12a) is connected, and a second fluid connection (15b) to which the second fluid line (12b) is connected,
the base body additionally comprising a film (17), which is arranged on the base body exposure surface (16) and covers the channel structure,
the film (17) sealing the channel structure against the egress of fluid onto the base body exposure surface (16).

10. The modular irradiation device according to the preceding claim, wherein the channel structure includes a multitude of the channels, which converge at the respective ends thereof in the respective fluid connection (15a, 15b),wherein preferably the channels at their ends respectively converge in pairs into combined channels, and the combined channels, in turn, converge in each case in pairs into combined channels until exactly two combined channels converge into one of the fluid connections (15a, 15b).

11. The modular irradiation device according to any one of claims 9 to 10, wherein the base body is a monolithic block, preferably an injection-molded part, into which the channel structure is embossed and/or cut,and/or wherein the film has a thickness of smaller than or equal to 80 µm, preferably of smaller than or equal to 60 µm, and/or of greater than or equal to 1 µm, preferably of greater than or equal to 10 µm and/or is a polyethylene film,and/or wherein a depth of the at least one channel is smaller than or equal to 300 µm, preferably smaller than or equal to 200 µm and/or preferably greater than or equal to 10 µm, preferably greater than or equal to 50 µm.

12. An irradiation method for irradiating a fluid, wherein
in a modular irradiation device according to any one of the preceding claims
at least one of the at least one carrier cassettes (2) being inserted into the main module (1),
thereafter the exposure surface (4) of the at least one carrier cassette (2) being irradiated with ionizing radiation, while a fluid to be irradiated is moved through the at least one irradiation line (13), and thereafter the carrier cassette (2) being removed from the main module (1).

13. The irradiation method according to the preceding claim, wherein at least one further carrier cassette is inserted into the main module (1) once or several times after the respective preceding carrier cassette (2) has been removed from the main module (1), and then the exposure surface (4) of the at least one further carrier cassette (2) is irradiated with ionizing radiation, while a fluid to be irradiated is moved through the at least one irradiation line (13) of this further carrier cassette (2).

14. The irradiation method according to either one of the two preceding claims,
wherein a disinfecting agent is moved through the channel structure of the fluid chip (14) after the at least one carrier cassette (2) has been inserted into the main module (1), and before the fluid to be irradiated is moved through the irradiation line (13), and/orwherein a cell medium is moved into the channel structure after the at least one carrier cassette (2) has been inserted into the main module, (1) and before the fluid to be irradiated is moved through the irradiation line (13), and/or after the fluid to be irradiated was moved through the channel structure.

15. The irradiation method according to any one of claims 12 to 14, wherein the fluid transport through the irradiation line (13) is effectuated in that the corresponding of the pumps (5a, 5b, 5c, 5d, 5e) generates negative pressure, and/orwherein the fluid to be irradiated comprises or is a cell suspension, virus suspension, medium, serum and/or blood sample,and/or wherein the ionizing radiation is electrons or UV radiation.

## Revendications

1. Dispositif d'irradiation modulaire, présentant
un module principal (1)
et au moins une cassette de support (2),
dans lequel la cassette de support (2) présente ce qui suit :
une surface d'exposition (4) sur laquelle s'étend au moins une conduite d'irradiation (13), dans lequel un fluide à irradier peut être conduit dans la au moins une conduite d'irradiation (13),
ainsi qu'au moins une pompe (5a, 5b, 5c, 5d, 5e) qui est raccordée à la au moins une conduite d'irradiation (13) par l'intermédiaire d'une première conduite de fluide, et
dans lequel le module principal (1) présente ce qui suit :
au moins un logement (3) dans lequel la au moins une cassette de support (2) peut être insérée de manière à pouvoir être retirée sans être détruite, ainsi que
au moins un actionneur de pompe (6a, 6b) qui est disposé de telle sorte que la au moins une pompe (5a, 5b, 5c, 5d, 5e) peut être actionnée lorsque la au moins une cassette de support (2) est insérée dans le logement (3) correspondant.

2. Dispositif d'irradiation modulaire selon la revendication précédente, dans lequel la cassette de support (2) présente au moins une autre des pompes (5a, 5b, 5c, 5d, 5e) raccordée à la conduite d'irradiation (13) par l'intermédiaire d'une deuxième conduite de fluide, et/ou dans lequel l'actionneur de pompe (6a, 6b) présente un élément de couplage de pompe qui est disposé de sorte qu'il vient en prise par coopération de forme avec un élément mobile de la au moins une pompe (5a, 5b, 5c, 5d, 5e) correspondante lorsque la cassette (2) est insérée dans le module principal (1).

3. Dispositif d'irradiation modulaire selon l'une quelconque des revendications précédentes, dans lequel
la au moins une cassette de support (2) présente au moins une soupape (9a, 9b, 9c) disposée dans au moins l'une des conduites de fluide (12a, 12b), avec laquelle un écoulement de fluide peut être contrôlé entre la pompe (5a, 5b, 5c, 5d, 5e) correspondante, qui est raccordée à la conduite d'irradiation (13) par l'intermédiaire de cette conduite de fluide (12a, 12b), et la conduite d'irradiation (13), et
dans lequel le module principal (1) présente, pour une, plusieurs ou toutes des au moins une soupapes (9a, 9b, 9c) respectivement un actionneur de soupape (8a, 8b) qui est ou sont respectivement disposés de telle sorte que la soupape (9a, 9b, 9c) correspondante peut être réglée par eux lorsque la au moins une cassette de support (2) est disposée dans le logement (3) correspondant.

4. Dispositif d'irradiation modulaire selon la revendication précédente, dans lequel la au moins une soupape (9a, 9b, 9c) présente un robinet avec lequel elle peut être réglée pour commander l'écoulement de fluide,
dans lequel l'actionneur de soupape (8a, 8b) correspondant présente un élément de couplage qui peut être couplé au robinet de telle sorte qu'avec l'actionneur de soupape (8a, 8b), une force de réglage de la soupape (9a, 9b, 9c) ou un couple de réglage de la soupape (9a, 9b, 9c) peut être exercé(e) sur le robinet ; et
dans lequel l'élément de couplage est disposé de sorte qu'il vient en prise avec le robinet lorsque la cassette de support (2) est insérée dans le module principal (1), et/ou dans lequel la au moins une soupape (9a, 9b, 9c) présente ou est une soupape à trois voies avec trois raccordements, dans lequel l'une des conduites de fluide (12a, 12b) est raccordée à un premier des raccordements, la au moins une pompe (5a, 5b, 5c, 5d, 5e) est raccordée à un deuxième des raccordements et une autre des pompes (5a, 5b, 5c, 5d, 5e) est raccordée au troisième des raccordements.

5. Dispositif d'irradiation modulaire selon l'une quelconque des revendications précédentes, dans lequel au moins une pompe (5a, 5b, 5c, 5d, 5e) présente une chambre de fluide et un piston, dans lequel le piston ferme la chambre de fluide de manière étanche au fluide et peut être déplacé dans la chambre de fluide, dans lequel au moins un actionneur de pompe (6a, 6b) agit sur le piston de la pompe (5a, 5b, 5c, 5d, 5e) lorsque la au moins une cassette de support (2) est insérée dans le logement (3) correspondant et il est possible d'exercer une force avec l'actionneur de pompe (6a, 6b) dans une direction de déplacement du piston.

6. Dispositif d'irradiation modulaire selon l'une quelconque des revendications précédentes, dans lequel au moins une pompe (5a, 5b, 5c, 5d, 5e) est une seringue, de préférence une seringue en plastique remplaçable, et/ou dans lequel la cassette de support (2) présente trois pompes (5a, 5b, 5c, 5d, 5e) raccordées à la deuxième conduite de fluide (12a, 12b) et deux pompes (5a, 5b, 5c, 5d, 5e) raccordées à la première conduite de fluide (12a, 12b).

7. Dispositif d'irradiation modulaire selon l'une quelconque des revendications précédentes,
dans lequel le module principal (1) présente une surface de module principal (10), et dans lequel la surface d'exposition (11) de la cassette de support (2) et la surface de module principal (10) sont coplanaires lorsque la cassette de support (2) est insérée dans le logement (3) correspondant du module principal (1), et/ou dans lequel
le module principal (1) présente exactement pour chaque pompe (5a, 5b, 5c, 5d, 5e) raccordée à l'une des conduites de fluide (12a, 12b), qui est de préférence utilisable comme dérivation pour le fluide, un actionneur respectif avec lequel la pompe (5a, 5b, 5c, 5d, 5e) correspondante peut être actionnée.

8. Dispositif d'irradiation modulaire selon l'une quelconque des revendications 2 à 7, dans lequel
le module principal (1) présente deux parties latérales (7a, 7b) mobiles et/ou rabattables,
dans lequel de préférence ceux des actionneurs permettant de déplacer les pompes (5a, 5b, 5c, 5d, 5e) raccordées par l'intermédiaire de la première conduite de fluide (12a, 12b) à la conduite d'irradiation (13) sont disposés sur l'une des parties latérales (7a, 7b), et dans lequel ceux des actionneurs avec lesquels les pompes (5a, 5b, 5c, 5d, 5e) raccordées à la conduite d'irradiation (13) par l'intermédiaire de la deuxième conduite de fluide (12a, 12b) sont mobiles sont disposés sur l'autre des parties latérales (7a, 7b).

9. Dispositif d'irradiation modulaire selon l'une quelconque des revendications précédentes, dans lequel
la cassette de support (2) présente une puce fluidique (14),
dans lequel la puce fluidique (14) présente un corps de base,
dans lequel le corps de base présente une surface d'exposition de corps de base (16) dans laquelle au moins une conduite d'irradiation (13) est réalisée, dans lequel la au moins une conduite d'irradiation (13) est une structure de canal avec au moins un canal,
dans lequel le corps de base présente en outre un premier raccord de fluide (15a) auquel est raccordée la première conduite de fluide (12a) et un deuxième raccord de fluide (15b) auquel est raccordée la deuxième conduite de fluide (12b),
dans lequel le corps de base présente en outre un film (17), lequel est disposé sur la surface d'exposition de corps de base (16) et recouvre la structure de canal,
dans lequel le film (17) étanchéifie la structure de canal contre toute fuite de fluide sur la surface d'exposition de corps de base (16).

10. Dispositif d'irradiation modulaire selon la revendication précédente, dans lequel la structure de canal présente une pluralité de canaux qui convergent à leurs extrémités respectives dans le raccord de fluide (15a, 15b) respectif, dans lequel de préférence les canaux convergent à leurs extrémités respectivement par paires en canaux communs et les canaux communs convergent à leur tour respectivement par paires en canaux communs jusqu'à ce qu'exactement deux canaux communs convergent dans l'un des raccords de fluide (15a, 15b).

11. Dispositif d'irradiation modulaire selon l'une quelconque des revendications 9 à 10, dans lequel le corps de base est un bloc monolithique, de préférence une pièce moulée par injection, dans lequel la structure de canal est estampée et/ou découpée, et/ou dans lequel le film a une épaisseur inférieure ou égale à 80 µm, de préférence inférieure ou égale à 60 µm et/ou supérieure ou égale à 1 µm, de préférence supérieure ou égale à 10 µm et/ou est un film de polyéthylène, et/ou dans lequel une profondeur d'au moins un canal est inférieure ou égale à 300 µm, de préférence inférieure ou égale à 200 µm et/ou de préférence supérieure ou égale à 10 µm, de préférence supérieure ou égale à 50 µm.

12. Procédé d'irradiation pour irradier un fluide, dans lequel, dans
un dispositif d'irradiation modulaire selon l'une quelconque des revendications précédentes
au moins une cassette de support (2) est insérée dans le module principal (1),
ensuite, la surface d'exposition (4) d'au moins une cassette de support (2) est irradiée avec un rayonnement ionisant tandis qu'un fluide à irradier est déplacé à travers au moins l'une conduite d'irradiation (13), et ensuite, la cassette de support (2) est retirée du module principal (1).

13. Procédé d'irradiation selon la revendication précédente dans lequel, une ou plusieurs fois après le retrait de la cassette de support (2) précédente du module principal (1), au moins une autre cassette de support est insérée dans le module principal (1), puis la surface d'exposition (4) d'au moins une autre cassette de support (2) est irradiée avec un rayonnement ionisant, tandis qu'un fluide à irradier est déplacé à travers la au moins une conduite d'irradiation (13) de cette cassette de support (2).

14. Procédé d'irradiation selon l'une des deux revendications précédentes,
dans lequel un désinfectant est déplacé à travers la structure de canal de la puce fluidique (14) après qu'au moins une cassette de support (2) a été insérée dans le module principal (1) et avant que le fluide à irradier soit déplacé à travers la conduite d'irradiation (13), et/ou dans lequel un milieu cellulaire est déplacé dans la structure de canal après qu'au moins une cassette de support (2) a été insérée dans le module principal (1) et avant que le fluide à irradier soit déplacé à travers la conduite d'irradiation (13) et/ou après que le fluide à irradier a été déplacé à travers la structure de canal.

15. Procédé d'irradiation selon l'une quelconque des revendications 12 à 14, dans lequel le transport de fluide à travers la conduite d'irradiation (13) est provoqué par le fait que la pompe correspondante parmi les pompes (5a, 5b, 5c, 5d, 5e) génère une dépression, et/ou dans lequel le fluide à irradier contient ou est une suspension cellulaire, une suspension virale, un milieu, un sérum et/ou un échantillon de sang, et/ou dans lequel le rayonnement ionisant est constitué d'électrons ou de rayonnement UV.
